# EUROPEAN PATENT SPECIFICATION

(11) **EP 0 531 119 B1**
(45) Date of publication and mention of the grant of the patent: **11.10.1995**
(21) Application number: 92307981.8
(22) Date of filing: 03.09.1992
(51) Int. Cl.: C07D 405/12, C07D 409/12, A61K 31/47, A61K 31/44, A61K 31/38

(54) **N,N',N'-trisubstituted -5-bis-aminomethylene-1,3-dioxane-4,6-dione inhibitors of acyl-CoA: cholesterol-acyl transferase**
N,N',N'-trisubstituierte 5-Bisaminomethylen-1,3-Dioxan-4,6-Dione als Acyl-CoA: Cholesterol-Acyltransferasehemmer
5-Bisaminométhylène-1,3-dioxane-4,6-diones N,N',N'-trisubstitués comme inhibiteurs d'acyl-CoA: cholesterol-acyl transférase

(30) Priority: 06.09.1991 US 755918; 06.03.1992 US 847127; 07.05.1992 US 879494; 22.07.1992 US 914886
(43) Date of publication of application: 10.03.1993
(73) Proprietor: AMERICAN HOME PRODUCTS CORPORATION, Madison, New Jersey 07940-0874 (US)
(72) Inventor: Fobare, William Floyd, Hamilton, New Jersey (US); Strike, Donald Peter, St. Davids, Pennsylvania 19087 (US); Andrae, Patrick Michael, Plainsboro, New Jersey 08536 (US)
(74) Representative: Wileman, David Francis, Dr.

(56) References cited:
- EP-A- 0 344 425
- EP-A- 0 471 493
- WISSENSCHAFTLICHE ZETISCHRIFT - MARTIN-LUTHER-UNIVERSIT[T HALLE-WITTENBERG, MATHEMATISCH-NATURWISSENSCHAFTLICHE REIHE vol. 38, no. 3, 1989, HALLE, DE pages 27 - 36 M. AUGUSTIN ET AL. 'Ringschlussreaktionen mit Keten-S,N bzw. -aminalen des 2,2-Dimethyl-1,3-dioxan-4,6-dions (Meldrums ure)'

## Description

### Background of the Invention

This invention relates to chemical compounds which display inhibition of Acyl-Coenzyme A: Cholesterol Acyltransferase (ACAT). Compounds of this type aid in reducing cholesterol absorption and its effect on atherosclerosis.

Atherosclerosis is the most common form of arteriosclerosis and is characterized by the buildup of phospholipids and esterified cholesterol in large and medium arteries causing them to be inelastic and thus weakened. These inelastic and occluded arteries are the most common cause of ischemic heart disease.

ACAT is an important enzyme for the intracellular esterification of cholesterol. Studies of this enzyme in cultured cells (M.S. Brown, J. Biol. Chem. 1980, 255, 9344) has shown that increases in ACAT activity represent increases in the presence of cholesterol laden lipoproteins. Regulation of ACAT helps prevent the absorption of cholesterol in the intestinal mucosa, and assists in the reversal of already present atherosclerotic lesions.

EP-A-344425 discloses the use of certain N-[[(2,6-disubstituted)phenyl]-N'-arylalkyl] ureas as cholesterol acyltransferase inhibitors.

### Description of the Invention

In accordance with this invention, there is provided a group of diaminomethylene dioxane dione derivatives of formula 1: 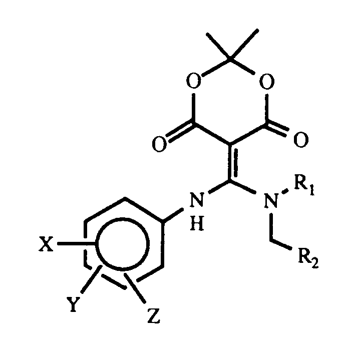
in which
- X, Y and Z: are, independently, hydrogen, halogen, hydroxy, nitro, cyano, carboxyl, trifluoromethyl, phenyl, amino, alkylamino of 1 to 12 carbon atoms, dialkylamino in which each alkyl group has 1 to 12 carbon atoms, alkyl of 1 to 12 carbon atoms or alkoxy of 1 to 12 carbon atoms;
- R₁: is hydrogen,alkyl of 1 to 18 carbon atoms, alkenyl of 2 to 18 carbon atoms, cycloalkyl of 5 to 8 carbon atoms, phenyl, benzyl or substituted phenyl or benzyl where the substituents are alkyl of 1 to 12 carbon atoms or alkoxy of 1 to 12 carbon atoms;
- R₂: is 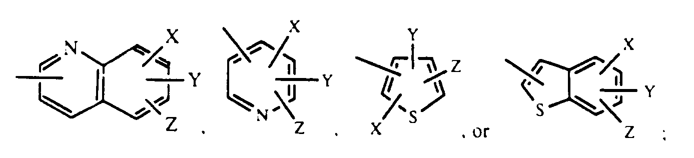 in which X, Y and Z independently have a value as defined above for X, Y and Z, the same or different; or a pharmaceutically acceptable salt thereof.

The halogen substituent referred to above may be chlorine, bromine, fluorine or iodine, fluorine being preferred. The pharmaceutically acceptable salts are derived from known inorganic or organic acids such as hydrochloric, hydrobromic, sulfuric, nitric, phosphoric, methanesulfonic, ethanesulfonic, hydroxyethanesulfonic, toluene sulfonic, naphthalenesulfonic, formic, acetic, propionic, oxalic, succinic, glycollic, lactic, malic, tartaric, citric, ascorbic, maleic, hydroxymaleic, pyruvic, phenylacetic, benzoic, para-amino benzoic, para-hydroxybenzoic, salicylic, sulfanilic acids, and the like.

Of these compounds, those preferred of the quinolinyl substituted series, on the basis of their in vitro and in vivo potency are those of formula 3: 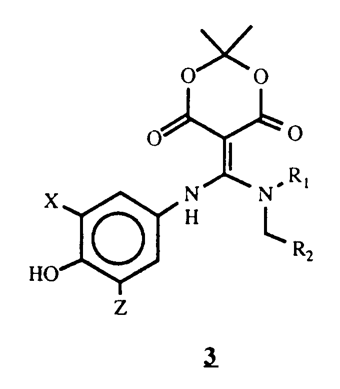
in which
X and Z are, independently, alpha branched alkyl of 1 to 6 carbon atoms;
R₁ is alkyl of 1 to 18 carbon atoms;
and
R₂ is 2-, 3- or 4-quinolinyl;
or a pharmaceutically acceptable salt thereof.

Those preferred in the pyridinyl substituted series ,on the basis of their in vitro and in vivo potency are those of formula **4**: 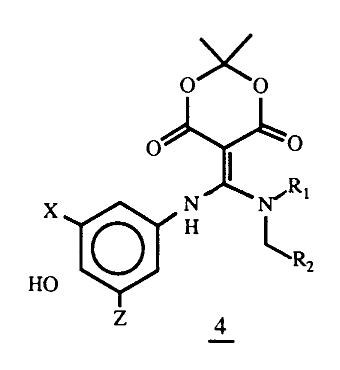
in which
X and Z are, independently, alpha branched alkyl of 1 to 6 carbon atoms;
R₁ is alkyl of 1 to 18 carbon atoms;
and
R₂ is 2-, 3- or 4- pyridinyl;
or a pharmaceutically acceptable salt thereof.

The preferred compounds containing the thienyl substituents and those of formula 5: 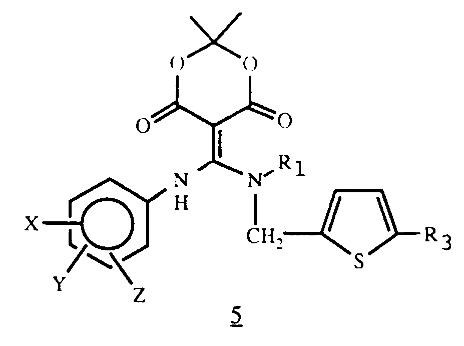
in which
- X, Y and Z: are, independently, hydrogen, alkyl of 1 to 6 carbon atoms, alkoxy of 1 to 6 carbon atoms, or hydroxy; more preferrably, the alkyl groups have 1 to 4 carbon atoms and the alkoxy groups have 1 to 3 carbon atoms;
- R₁: is alkyl of 6 to 10 carbon atoms or cycloalkyl of 5 to 7 carbon atoms, most preferrably R₁ is alkyl of 6 to 10 carbon atoms, optionally alpha branched;
and R₃ is alkyl of 1 to 6 carbon atoms, more preferrably R₃ is branched chain alkyl of 3 to 6 carbon atoms.

The preferred compounds containing the benzothienyl substituents are those of formula 6: 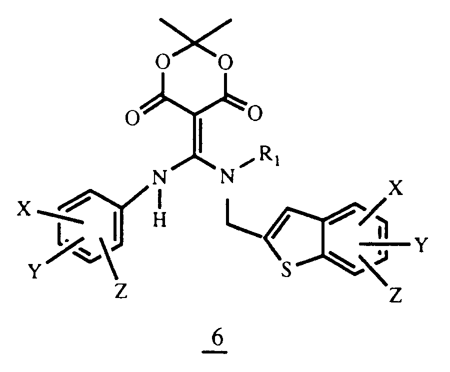
in which
X, Y and Z are, independently alkyl of 1 to 4 carbon atoms, alkoxy of 1 to 4 carbon atoms or hydroxy; and
R₁ is alkyl of 1 - 18 carbon atoms; or a pharmaceutically acceptable salt thereof.

This invention also provides processes for preparing the compounds of formula **1** which comprise
a) reacting a compound of formula 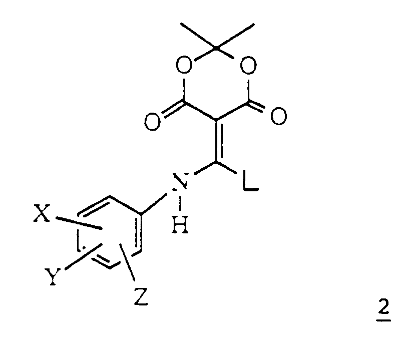 wherein X, Y and Z are as defined above and L is a leaving group, with an amine of formula HN(R₁)CH₂R₂ where R₁ and R₂ are as defined above;
   or
b) reacting 2,2-dimethyl-1,3-dioxane-4,6-dione (Meldrum's Acid) with a carbodiimide of formula 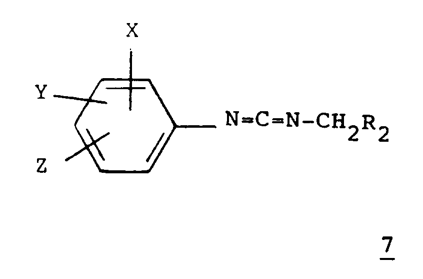 to give a compound of formula I as defined above wherein R₁ is hydrogen; and if desired after said process a) or b) converting any value of X, Y or Z to one of the other values for X, Y and Z by known methods and further if desired isolating the product as a pharmaceutically acceptable salt.

Examples of leaving groups for L in formula **2** are -SR and -S(O)R, where R is an organic radical e.g alkyl, aralkyl or aryl such as methyl, phenyl, tolyl or benzyl.

Compounds of formula **2** may be prepared by methods known in the literature, for example, when L is SR by reacting a compound of formula **8** 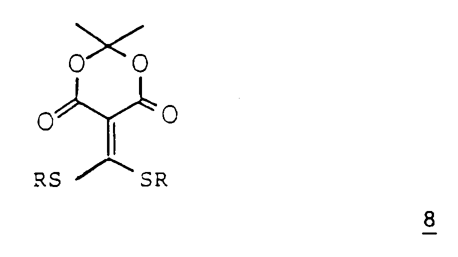
wherein R is an organic radical e.g as exemplified above, with an aniline of formula 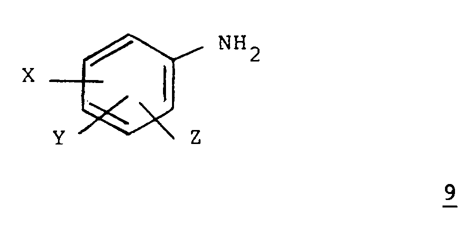
where X, Y and Z are as defined above.

Both reactions above may be conveniently carried out by mixing the reagents in the presence of an inert solvent with heating if necessary.

Compounds of formula **8** can be prepared by processes known in the art. For example, when L is SR where R is alkyl or aralkyl then compounds of formula can be prepared by reacting Meldrum's acid with CS₂ and an organic halide, e.g RI presence of a base such as triethylamine.

Oxidation of a compound of formula **2** wherein L is SR with a peracid e.g m-chloroperbenzoic acid give corresponding compounds where L is S(O)R.

The compounds of this invention are conveniently prepared by conversion of 2,2-dimethyl-1,3-dioxane-4,6-dione to the corresponding 5-bis-(methylthio) methylene derivative with carbon disulfide and methyl iodide in dimethylsulfoxide in the presence of a base such as triethylamine, followed by sequential displacement of the methylthio groups with the desired amines, thusly: 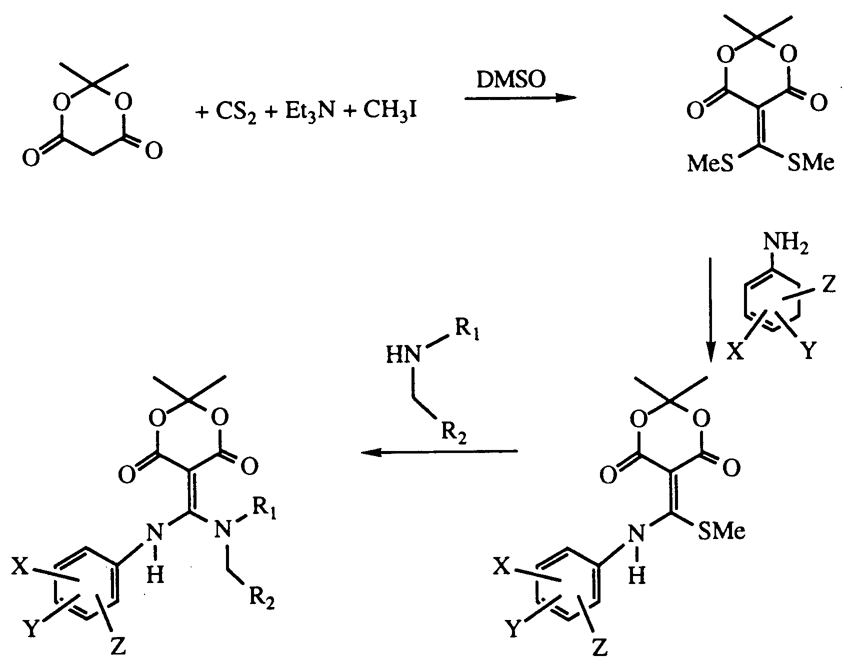
The following examples illustrate without limitation the preparation of representative compounds of this invention.

### Method A

### Example 1

### 5-[[[3,5-Bis(1,1-dimethylethyl)-4-hydroxyphenyl]amino][hexyl(4-quinolinylmethyl)amino]methylene]-2,2-dimethyl-1,3-dioxane-4,6-dione

### Procedure 1

To a solution of 6.4 g (25.8 mmol) of 5-[bis(methylthio)methylene]-2,2-dimethyl-1,3-dioxane-4,6-dione and 4.84 g (56.0 mmol) of sodium bicarbonate in 10 mL of degassed DMSO was added 10.0 g (36.0 mmol) of 3,5-di-t-butyl-4-hydroxyaniline hydrochloride in 30 mL of degassed DMSO over a 5 hour period at room temperature. Stirring was continued for an additional 19 hours. The reaction mixture was poured into cold H₂O and the product filtered. The solid was dried and dissolved in ethyl acetate and filtered again. The solvent was removed at reduced pressure and the residue submitted to a column chromatography on silica gel (3:1 to 2:1 hexane-ethyl acetate) to yield 9.8 g (90%) of a solid that was used without further purification.

### Procedure 2

To a solution of 3.05 mL (98.8 mmol) of hexylamine and 16.5 mL (33 mmol) of 2N HCl in 25 mL of methanol was dissolved 2.59 g (16.5 mmol) of 4-quinolinecarboxaldehyde. To this mixture was added 0.62 g (9.9 mmol) of sodium cyanoborohydride. After 1 hour the pH was lowered to 7.8 with 2N HCl. This stirred at room temperature for 1 hour. The solvents were removed at reduced pressure and the residue was added to 30 mL of H₂O and the solution was acidified to pH2 with concentrated HCl. This solution was washed 3 X 50 mL of diethyl ether and then it was made basic with NaOH to pH12. The aqueous solution was extracted 3 times with 50 mL of CHCl₃ which extracts were combined, dried (Na₂SO₄) and the solvent removed at reduced pressure. Column chromatography of the residue on silica gel (90:10 ethyl acetate - hexane to 5:95 triethylamine-ethyl acetate) yielded 2.5 g (64%) of an oil which was used without further purification.

### Procedure 3

To a solution of 1.2 g (2.86 mmol) of the compound from Method A, Procedure 1 in 30 mL of acetonitrile was added 0.73 g (3.0 mmol) of the amine from Method A, Procedure 2, 0.4 mL (2.86 mmol) of triethylamine and 0.47 g (1.57 mmol) of mercuric sulfate. The mixture was allowed to stir at reflux for 4 hours. The solution was cooled, diluted with ethyl acetate and filtered through Celite®. The solvents were removed at reduced pressure and column chromatography of the residue on silica gel (80:20 ethyl acetate - hexanes to 100% ethyl acetate) yielded 0.52 g (46%) of a yellow powder. This was recrystallized from ethyl acetate - hexanes to yield the title compound as a yellow solid (mp 127-133°C). IR (KBr): 3420, 3222, 2950, 2868, 1612 1565, 1507, 1462, 1429, 1381, 1360, 1251, 1229, 1198, 1161, 1113, 1089, 1021, 922, 883, 782 and 762 cm⁻¹. ¹H NMR (400 MHz, CDCl₃): δ 9.60 (br s, 1H), 8.90 (d, 1H, J = 4.48 Hz), 8.16 (d, 1H, J = 8.36), 7.74-7.61 (m, 3H), 7.55 (t, 1H, J = 7.28 Hz), 6.71 (s, 2H), 5.22 (s, 1H), 4.90 (s, 2H), 3.23 (br s, 2H), 1.67 (br s, 8H), 1.22 (br s, 24H), 0.83 (t, 3H, J = 6.76 Hz).

| Elemental analysis for C₃₇H₄₉N₃O₅ | | | |
|---|---|---|---|
| Calc'd: | C, 72.17; | H, 8.02; | N, 6.82 |
| Found: | C, 71.78; | H, 8.00; | N, 6.60 |

### Method B

### Example 2

### 5-[[(2,4-Dimethoxyphenyl)amino][hexyl(4-quinolinylmethyl)amino]methylene]-2,2-dimethyl-1,3-dioxane-4,6-dione

### Procedure 1

To a solution containing 2.0 g (8.05 mmol) of 5-[bis(methylthio)methylene]-2,2-dimethyl-1,3-dioxane-4,6-dione 1 in 40 mL of t-butanol, was added 1.23 g (8.05 mmol) of 2,4-dimethoxyaniline. The reaction mixture was allowed to stir at reflux for 24 hours. The mixture was cooled to room temperature and diluted with hexanes. The solid was filtered and used without further purification. Isolated: 2.3 g, 81% yield.

### Procedure 2

To a solution of 0.84 g (2.38 mmol) of the product from Method B, Procedure 1, was added 30 mL of acetonitrile, 0.33 g (1.31 mmol) of mercuric sulfate, 0.33 mL (2.38 mmol) of triethylamine and 0.64 g (2.6 mmol) of the amine synthesized in Method A, Procedure 2. The reaction mixture was allowed to stir at reflux for 1.5 hours. The mixture was then cooled to room temperature, diluted with ethyl acetate and filtered through Celite®. The solvent was removed at reduced pressure and column chromatography of the residue on silica gel (ethyl acetate to 90:10 ethyl acetate - ethanol) yielded a solid which after recrystallization from diisopropyl ether yielded 1.08 g (83%) of the title compound as a white solid (mp 144-147°C). IR (KBr): 3420, 2930, 2855, 1700, 1633, 1571, 1517, 1464, 1386, 1352, 1312, 1204, 1160, 1088, 1031 and 930 cm⁻¹. ¹H NMR (400 MHz, CDCl₃): δ 8.90 (d, 1H, J = 4.36 Hz), 8.8 (br s, 1H exchangeable), 8.17 (d, 1H, J = 8.08 Hz) , 7.76-7.66 (m, 3H), 7.54 (t, 1H, J = 7.28 Hz), 6.91 (d, 1H, J = 8.52 Hz), 6.33 (d, 1H, J = 2.48 Hz), 6.26 (dd, 1H, J = 8.52, 2.52 Hz), 4.94 (br s, 2H), 3.75 (s, 3H), 3.58 (s, 3H), 3.26 (br s, 2H), 1.61 (br s, 8H), 1.25 (br s, 6H), 0.83 (t, 3H, J = 6.84 Hz).

| Elemental analysis for C₃₁H₃₇N₃O₆ | | |
|---|---|---|
| Calc'd: | C, 67.99; | H, 6.81 |
| Found: | C, 67.84; | H, 6.91 |

### Example 3

### 5-[[[3,5-Bis(1,1-dimethylethyl)-4-hydroxyphenyl]amino][hexyl(2-quinolinylmethyl)amino]methylene]-2,2-dimethyl-1,3-dioxane-4,6-dione

This compound was synthesized using the methodology described in Method A except 2-quinolinecarboxaldehyde was substituted for 4-quinolinecarboxaldehyde to yield the title compound as a white solid (mp 161-163°C).

| Elemental analysis for C₃₇H₄₉N₃O₅ | | |
|---|---|---|
| Calc'd: | C, 72.17; | H, 8.02 |
| Found: | C, 71.91; | H, 8.09 |

### Example 4

### 5-[[(2,4-Dimethoxyphenyl)amino][hexyl(2-quinolinylmethyl)amino]methylene]-2,2-dimethyl-1,3-dioxane-4,6-dione

This compound was synthesized using the methodology described in Method B except 2-quinolinecarboxaldehyde was substituted for 4-quinolinecarboxaldehyde to yield the title compound as a light brown solid (mp 106-109°C).

| Elemental analysis for C₃₁H₃₇N₃O₆ | | |
|---|---|---|
| Calc'd: | C, 67.99; | H, 6.81 |
| Found: | C, 67.54; | H, 6.82 |

### Example 5

### 5-[[(2,4-Dimethoxyphenyl)amino]-[1-methylhexyl)(2-quinolinylmethyl)amino]methylene]-2,2-dimethyl-1,3-dioxane-4,6-dione

This compound was synthesized using the methodology described in Method A except 2-aminoheptane was used in place of hexylamine and 2-quinolinecarboxaldehyde was used instead of 4-quinolinecarboxaldehyde to yield the title compound as a solid (mp 148-151°C).

| Elemental analysis for C₃₂H₃₉N₅O₆ | | | |
|---|---|---|---|
| Calc'd: | C, 58.43; | H, 7.00; | N, 7.48 |
| Found: | C, 58.10; | H, 7.13; | N, 7.29 |

### Example 6

### 5-[[[3,5-Bis(1,1-dimethylethyl)-4-hydroxyphenyl]amino][1-methylhexyl)(2-quinolinylmethyl)amino]methylene]-2,2-dimethyl-1,3-dioxane-4,6-dione

This compound was synthesized using the methodology described in Method A except 2-aminoheptane was used in place of hexylamine and 2-quinolinecarboxaldehyde was used instead of 4-quinolinecarboxaldehyde to yield the title compound as a solid (mp 124-128°C).

| Elemental analysis for C₃₈H₅₁N₃O₅ | | | |
|---|---|---|---|
| Calc'd: | C, 72.47; | H, 8.16; | N, 6.67 |
| Found: | C, 72.16; | H, 8.04; | N, 6.29 |

### Example 7

### 5-[[(2,4-Dimethoxyphenyl)amino][(1-methylhexyl)(4-quinolinylmethyl)amino]methylene]-2,2-dimethyl-1,3-dioxane-4,6-dione

This compound was synthesized using the same methodology described in Method B except that 2-aminoheptane was substituted for hexylamine to yield the title compound as a solid (mp 197-202°C).

| Elemental analysis for C₃₂H₃₉N₃O₆ | | | |
|---|---|---|---|
| Calc'd: | C, 68.43; | H, 7.00; | N, 7.48 |
| Found: | C, 68.41; | H, 6.89; | N, 7.48 |

### Example 8

### 5-[[[3,5-Bis(1,1-dimethylethyl)-4-hydroxyphenyl]amino][(1-methylhexyl)(4-quinolinylmethyl)amino]methylene]-2,2-dimethyl-1,3-dioxane-4,6-dione

This compound was synthesized using the same methodology described in Method A except that 2-aminoheptane was substituted for hexylamine to yield the title compound as a solid (mp 147-151°C).

| Elemental analysis for C₃₈H₅₁N₃O₅ | | | |
|---|---|---|---|
| Calc'd: | C, 72.46; | H, 8.16; | N, 6.67 |
| Found: | C, 72.11; | H, 8.21; | N, 6.35 |

### Example 9

### 5-[[[3,5-Bis(1,1-dimethylethyl)-4-hydroxyphenyl]amino][hexyl(3-quinolinylmethyl)amino]methylene]-2,2-dimethyl-1,3-dioxane-4,6-dione

This compound was synthesized using the methodology described in Method A except 3-quinolinecarboxaldehyde was substituted for 4-quinolinecarboxaldehyde to yield a light orange powder (mp 130-136°C).

| Elemental analysis for C₃₇H₄₉N₃O₅ | | | |
|---|---|---|---|
| Calc'd: | C, 72.17; | H, 8.02; | N, 6.82 |
| Found: | C, 71.74; | H, 8.07; | N, 6.81 |

### Method C

### Example 10

### 5-[(3,5-Di-tert-butyl-4-hydroxy-phenylamino)-(hexyl-(pyridin-4-ylmethyl)-amino)-methylene]-2,2-dimethyl-1,3-dioxane-4,6-dione

### Procedure 1

To a solution of 6.4 g (25.8 mmol) of 5-[bis(methylthio)methylene]-2,2-dimethyl-1,3-dioxane-4,6-dione and 4.84 g (56.0 mmol) of sodium bicarbonate in 10 mL of degassed DMSO was added 10.0 g (36.0 mmol) of 3,5-di-t-butyl-4-hydroxyaniline hydrochloride in 30 mL of degassed DMSO over a 5 hour period at room temperature. Stirring was continued for an additional 19 hours. The reaction mixture was poured into cold H₂O and the product filtered. The solid was dried and dissolved in ethyl acetate and filtered again. The solvent was removed at reduced pressure and the residue submitted to a column chromatography on silica gel (3:1 to 2:1 hexane-ethyl acetate) to yield 9.8 g (90%) of a solid that was used without further purification.

### Procedure 2

To a solution of 9.9 gm (91.85 mmol) of 4-aminomethylpyridine in 40 mL of methanol was added HCl saturated methanol to pH = 7. Then 4.8 g (39.9 mmol) of hexaldehyde was added followed by 1.65 g (26.3 mmol) of sodium cyanoborohydride at room temperature. After stirring for 72 hours the mixture was acidified to pH = 2 with concentrated HCl and the solvent was removed at reduced pressure. The residue was combined with 20 mL of H₂O and extracted 3 times with 75 mL of diethyl ether. The aqueous layer was made basic (pH = 14) with solid NaOH and extracted 3 times with 80 mL of ethyl acetate. The combined ethyl acetate layers were dried (Na₂SO₄) and the solvent removed at reduced pressure. Column chromatography of the residue on 300 g of silica gel (4% MeOH-CHCl₃) yielded 3.5 g (46%) of the amine which was used without further purification or characterization.

### Procedure 3

To a solution of 0.97 g (2.3 mmol) of the material from Method C, Procedure 1 in 20 mL of CH₃CN was added 0.46 g (2.4 mmol) of the amine from Method C, Procedure 2, 0.32 g (2.3 mmol) of Et₃N and 0.41 g (1.38 mmol) of HgSO₄. The reaction mixture was allowed to reflux for 3.5 hours. The mixture was cooled to room temperature, filtered through Celite® and the solvents removed at reduced pressure. Column chromatography (1% ethanol-ethyl acetate to 3% ethanol-ethyl acetate) on 150 g of silica gel yielded 0.97 g of the title compound as a solid (mp 137-140°C) after recrystallization (isopropyl ether-ethyl acetate-hexanes). IR (KBr) 3260, 1952, 1868, 1625, 1432, 1386, 1362, 1242, 1199, 1161, 1114, 1086, 924, 783 and 733 cm^{-1.} ¹HNMR (400 MHz, CDCl₃): δ 9.61 (br s, 1H), 8.53 (d, 2H, J = 4.9 Hz), 7.32 (br s, 2H) 6.79 (s, 2H), 5.29 (s, 1H), 4.28 (br s, 2H), 3.23 (br s, 2H), 1.65-1.57 (m, 8H), 1.38 (s, 18H), 1.20 (s, 6H), 0.83 (t, 3H, J = 6.57 Hz).

| Elemental analysis for C₃₃H₄₇N₃O₅ | | | |
|---|---|---|---|
| Calc'd: | C, 70.06; | H, 8.37; | N, 7.43 |
| Found: | C, 70.01; | H, 8.39; | N, 7.08 |

### Method D

### Example 11

### 5-[[(2,4-Dimethoxyphenylamino)-[hexyl-(pyridin-4-ylmethyl)-amino]-methylene]-2,2-dimethyl-[1,3]-dioxane-4,6-dione

### Procedure 1

To a solution containing 2.0 g (8.05 mmol) of 5-[bis(methylthio)methylene]-2,2-dimethyl-1,3-dioxane-4,6-dione in 40 mL of t-butanol, was added 1.23 g (8.05 mmol) of 2,4-dimethoxyaniline. The reaction mixture was cooled to room temperature and diluted with hexanes. The solid was filtered and used without further purification. Isolated: 2.3 g, 81% yield.

### Procedure 2

To a solution of 0.93 g (2.61 mmol) of the product from Method D, Procedure 1, in 15 mL of CH₃CN was added 0.33 g (2.7 mmol) of the amine from Method C, Procedure 2, 0.46 g (1.37 mmol) of HgSO₄ and 0.36 mL (2.6 mmol) of Et₃N. The reaction mixture was allowed to reflux for 17 hours. The mixture was cooled then diluted with ethyl acetate and filtered through Celite®. The solvents were removed at reduced pressure. The residue was chromatographed on silica gel (3% MeOH-CHCl₃). The oil obtained was crystallized (isopropylether-ethyl acetate-hexanes) to yield 1.01 g (78%) of the title compound as a solid (mp 148-150°C). IR (KBr): 3440, 3220, 2992 2932, 2858, 1689, 1621, 1509, 1461, 1434, 1413, 1382, 1347, 1309, 1260, 1204, 1157, 1132, 1080, 1028, 926, 829, 783 and 724 cm⁻¹. ¹H NMR (400 MHz, CDCl₃): δ 9.01 (br s, 1H), 8.56 (d, 2N, J = 5.92 Hz), 7.36 (d, 2H, J = 5.07 Hz), 7.04 (d, 1H, J = 8.45 Hz), 6.46 (m, 2H), 4.35 (br s, 2H), 3.82 (s, 3H), 3.73 (s, 3H), 3.23 (t, 2H, J = 6.88 Hz), 1.64-1.51 (m, 8H), 1.20 (br s, 6H), 0.85 (t, 3H, J = 6.78 Hz).

| Elemental analysis for C₂₇H₃₅N₃O₆ | | | |
|---|---|---|---|
| Calc'd: | C, 65.17; | H, 7.09; | N, 8.44 |
| Found: | C, 65.18; | H, 7.25; | N, 8.29 |

### Example 12

### 5-[(3,5-Di-tert-butyl-4-hydroxy-phenylamino)-(hexyl-(pyridin-2-ylmethyl)-amino]-methylene]-2,2-dimethyl-[1,3]-dioxane-4,6-dione

This compound was synthesized using the procedure in Method C except 2-aminomethylpyridine was used instead of 4-aminomethylpyridine to yield (91%) a solid (mp 169-170°C).

| Elemental analysis for C₃₃H₄₇N₃O₅ | | | |
|---|---|---|---|
| Calc'd: | C, 70.06; | H, 8.34; | N, 7.43 |
| Found: | C, 70.05; | H, 8.47; | N, 6.98 |

### Example 13

### 5-[(2,4-Dimethoxyphenylamino)-[hexyl-(pyridin-2-ylmethyl)-amino]-methylene]-2,2-dimethyl-[1,3]-dioxane-4,6-dione

This compound was synthesized using the procedure in Method D except 2-aminomethyl- pyridine was used instead of 4-aminomethylpyridine to yield(82%) a solid (mp 104-105°C).

| Elemental analysis for C₂₇H₃₅N₃O₆ | | | |
|---|---|---|---|
| Calc'd: | C, 65.17; | H, 7.09; | N, 8.44 |
| Found: | C, 64.92; | H, 7.08; | N, 8.22 |

### Example 14

### 5-[(2,4-Dimethoxy-phenylamino)-[hexyl-(pyridin-3-ylmethyl)-amino]-methylene]-2,2-dimethyl-[1,3]-dioxane-4,6-dione

This compound was synthesized using the procedure in Method D except 3-aminomethyl- pyridine was used instead of 4-aminomethylpyridine to yield (95%) a solid (mp 154-155°C).

| Elemental analysis for C₂₇H₃₅N₅O₆ | | | |
|---|---|---|---|
| Calc'd: | C, 65.17; | H, 7.09; | N, 8.44 |
| Found: | C, 64.87; | H, 7.12; | N, 8.30 |

### Example 15

### 5-[(3,5-Di-tert-butyl-4-hydroxy-phenylamino)-[hexyl-(pyridin-3-ylmethyl)-amino]-methylene]-2,2-dimethyl-1,3-dioxane-4,6-dione

This compound was synthesized using the procedure in Method C except 3-aminomethylpyridine was used instead of 4-aminomethylpyridine to yield (78%) a solid (mp 134-136°C).

| Elemental analysis for C₃₃H₄₇N₃O₅ | | | |
|---|---|---|---|
| Calc'd: | C, 70.06; | H, 8.37; | N, 7.43 |
| Found: | C, 70.25; | H, 8.57; | N, 7.09 |

### METHOD E

### Example 16

### 5-[[(2,4-Dimethoxyphenyl)amino][[[5-(2,2-dimethylpropyl)-2-thienyl]methyl]heptylamino]-methylene]-2,2-dimethyl-1,3-dioxane-4,6-

### Procedure 1

To a solution of 50.4 g (0.6 mol) of thiophene and 72.3 g (0.6 mol) of pivaloyl chloride in 500 mL of benzene at 0°C was added 70.2 mL (0.6 mol) of SnCl₄ over a 0.75 hour period. The solution stirred at 0°C for 0.5 hours then at room temperature for 2 hours. The reaction was quenched with 100 mL of 10% HCl and the organic layer was separated, washed twice with H₂O, then dried (MgSO₄) and the solvents were removed at reduced pressure. Distillation under vacuum (1.2 mm Hg) at 71-73°C yielded 67.0 g (55%) of an oil. IR (film) 3090, 2975, 1640, 1481, 1411, 1363, 1347, 1276, 1178, 1059, 908, 851 and 718 cm⁻¹. ¹H NMR (200 MHz, CDCl₃): δ7.83 (d, 1H, J = 4.0 Hz), 7.62 (dd, 1H, J = 4.0, 3.8 Hz), 7.17 (dd, 1H, J = 4.0, 3.8 Hz), 1.43 (s, 9H).

### Procedure 2

To a solution of 67.0 g (0.4 mol) of ketone from Method E, Procedure 1 (thien-2-yl, tert-butyl ketone) in 250 mL of ethanol was added 48.0 mL (1.0 mol) hydrazine. The reaction mixture was allowed to reflux for 10 days. The solvents were then removed at reduced pressure. The residue was added to 300 mL of toluene and 45 g (0.4 mol) of potassium t-butoxide was added. The reaction was heated slowly (exothermic) then taken to reflux for 3.5 hours. The reaction mixture was cooled to room temperature and added to H₂O. The layers were separated and the aqueous layer was extracted with diethyl ether. The combined organic layers were dried (MgSO₄) and the solvents removed at reduced pressure. Distillation at 16-18 mm Hg yielded 60 gms (98%) of a liquid (B.P. 86-89°C). IR (film) 2942, 1478, 1469, 1425, 1387, 1357, 1232, 1194, 1178, 1107, 1072, 1039, 848, 819 and 682 cm⁻¹. ¹H NMR (80 MHz, CDCl₃): δ 7.30 (m, 3H), 2.65 (s, 2H), 0.98 (s, 9H).

### Procedure 3

To a solution of 11.0 mL (0.14 mol) of dimethylformamide in 15 mL of dichloroethane at 0°C was added 13.2 mL (0.14 mol) of phosphorous oxychloride over a 0.5 hour period. At 0°C a solution 20 g (0.13 mol) of the 2-neopentylthiophene (Method E, Procedure 2) in 40 mL of ethylene dichloride was added over a 1 hour period. The reaction mixture was allowed to warm to room temperature over 0.5 hours then to reflux for 2 hours. The solution was cooled when 96 g of NaOAc·3 H₂O in 200 mL of H₂O was added and stirred for 10 minutes. The layers were separated and the aqueous layer was extracted twice with diethyl ether. The combined organic layers were washed with saturated aqueous K₂CO₃, then dried (MgSO₄) and concentrated at reduced pressure. Distillation of the residue at 0.6 mm Hg yielded 19.6 g (83%) of an oil (BP. 107°C). This was used without further characterization.

### Procedure 4

To a solution of 19.6 g (0.11 mol) of the aldehyde from Method E, Procedure 3 (5-(2,2-dimethylpropyl)-2-thienyl-carboxaldehyde) in 70 mL of benzene was added 16.0 mL (0.11 mol) of 1-aminoheptane and a crystal of p-toluenesulfonic acid. The reaction mixture was allowed to reflux for 16 hours using a Dean-Stark trap. The solution was cooled to room temperature and the solvent was removed at reduced pressure. The residue was added to 250 mL of dry THF and HCl gas was bubbled in for 10 minutes. The mixture was cooled to 0°C and 6.72 g (0.11 mol) of sodium cyanoborohydride in 50 mL of methanol was slowly added. The reaction mixture was allowed to stir at 0°C for 0.5 hours then at room temperature for 17 hours. The mixture was poured into 150 mL of 0.5 N NaOH and extracted twice with diethyl ether. The combined organic layers were dried (Na₂SO₄) and condensed at reduced pressure. Distillation at 0.4 mm Hg yielded 24.7 g (82%) of an oil (B.P. 153-155°C). IR (film) 3075, 2928, 2854, 1460, 1362, 1238, 1111, 800 and 739 cm⁻¹. ¹H NMR (400 MHz, CDCl₃): δ 6.68 (d, 1H, J = 6.0 Hz), 6.54 (d, 1H, J = 6.0 Hz), 3.80 (s, 2H), 2.62 (m, 4H), 1.60 (br s, 1H), 1.48 (m, 2H), 1.28 (m, 8H), 0.94 (s, 9H), 0.88 (t, 3H, J = 5.8 Hz).

### Procedure 5

To a solution containing 2.0 g (8.05 mmol) of 5-[bis(methylthio)methylene]-2,2-dimethyl-1,3-dioxane-4,6-dione in 40 mL of t-butanol, was added 1.23 g (8.05 mmol) of 2,4-dimethoxyaniline. The reaction mixture was allowed to stir at reflux for 24 hours. The mixture was cooled to room temperature and diluted with hexanes. The solid was filtered and used without further purification. Isolated: 2.3 g, 81% yield.

### Procedure 6

To a solution of 0.62 g (1.75 mmol) of the compound from Method E, Procedure 5 in 10 mL of acetonitrile was added 0.49 g (1.75 mmol) of the amine from Method E, Procedure 4, 0.31 g (1.05 mmol) of HgSO₄ and 0.25 mL (1.75 mmol) of triethylamine. This reaction mixture was allowed to reflux for 18 hours. The mixture was cooled, diluted with ethyl acetate and filtered through Celite®. The solvent was removed at reduced pressure and the residue was chromatographed on silica gel (2:1 hexanes-ethyl acetate to 1:1 hexanes - ethyl acetate) to yield 0.73 g (71%) of the title compound as a solid (mp 69-72°C). IR (KBr) 3420, 3210, 2955, 2860, 1702, 1634, 1571, 1512, 1456, 1390, 1367, 1312, 1208, 1160, 1084, 1038, 932, and 890 cm⁻¹. ¹H NMR (400 MHz, CDCl₃): δ 7.06 (d, 1H, J = 8.72 Hz), 6.85 (d, 1H, J = 3.40 Hz), 6.61 (d, 1H, J = 3.40 Hz), 6.47 (d, 1H, J = 2.56 Hz), 6.34 (dd, 1H, J = 8.72, 2.56 Hz), 4.57 (s, 2H), 3.81 (s, 3H), 3.77 (s, 3H), 2.96 (m, 2H), 1.70 (br s, 6H), 1.55 (m, 2H), 1.17 (m, 8H), 0.95 (s, 9H), 0.84 (t, 3H, J = 6.87 Hz).

| Elemental analysis for C₃₂H₄₆N₂O₆S | | | |
|---|---|---|---|
| Calc'd: | C, 65.50; | H, 7.90; | N, 4.77 |
| Found: | C, 65.73; | H, 7.97; | N, 4.74 |

### METHOD F

### Example 17

### 5-[[[3,5-Bis(1,1-dimethylethyl)-4-hydroxyphenyl]amino][[[5-(2,2-dimethylpropyl)-2-thienyl]methyl]heptylamino]methylene]-2,2-dimethyl-1,3-dioxane-4,6-dione

### Procedure 1

To a solution of 6.4 g (25.8 mmol) of 5-[bis(methylthio)methylene]-2,2-dimethyl-1,3-dioxane-4,6-dione and 4.84 g (56.0 mmol) of sodium bicarbonate in 10 mL of degassed DMSO was added 10.0 g (36.0 mmol) of 3,5-di-t-butyl-4-hydroxyaniline hydrochloride in 30 mL of degassed DMSO over a 5 hour period at room temperature. Stirring was continued for an additional 19 hours. The reaction mixture was poured into cold H₂O and the product filtered. The solid was dried and dissolved in ethyl acetate and filtered again. The solvent was removed at reduced pressure and the residue submitted to a column chromatography on silica gel (3:1 to 2:1 hexane-ethyl acetate) to yield 9.8 g (90%) of a solid that was used without further purification.

### Procedure 2

To a solution of 0.58 g (2.08 mmol) of the amine synthesized in Method E, Procedure 4 in 20 mL of CH₃CN was added 0.84 g of the compound from Method F, Procedure 1, 0.29 g (0.99 mmol) of HgSO₄ and 0.2 g (1.98 mmol) of triethylamine. The solution was allowed to reflux for 18 hours. The reaction mixture was cooled to room temperature, diluted with ethyl acetate and filtered through Celite®. The solvents were removed at reduced pressure and the residue was chromatographed on silica gel (3:1 hexanes - ethyl acetate to 1:1 hexanes - ethyl acetate) to yield after recrystallization (hexanes - ethyl acetate) 1.0 g (77%) of the title compound as a pale yellow solid (mp 178-179°C). IR (KBr) 3405, 2950, 2859, 1696, 1623, 1573, 1462, 1432, 1383, 1361, 1233, 1204, 1115, 1088, 931 and 800 cm⁻¹. ¹H NMR (400 MHz, CDCl₃): δ 6.93 (s, 2H), 6.85 (d, 1H, J = 3.32 Hz), 6.59 (d, 1H, J = 3.32 Hz), 5.23 (br s, 1H), 4.56 (s, 2H), 3.05 (m, 2H), 2.62 (s, 2H), 1.72-1.55 (m, 8H), 1.38 (s, 18H), 1.34-1.09 (m, 8H), 0.94 (s, 9H), 0.85 (t, 3H, J = 6.9 Hz).

| Elemental analysis for C₃₈H₄₈N₂O₅S | | | |
|---|---|---|---|
| Calc'd: | C, 69.65; | H, 8.93; | N, 4.28 |
| Found: | C, 69.76; | H, 9.05; | N, 4.12 |

### Example 18

### 5-[[(2,4-Dimethoxyphenyl)amino][[[5-(2,2-dimethylpropyl)-2-thienyl]methyl](1-methylhexyl)-amino]methylene]-2,2-dimethyl-1,3-dioxane-4,6-dione

This compound was synthesized using the same methodology as in Method E except 2-aminoheptane was substituted for 1-aminoheptane to yield 1.37 g (81%) of a white powder (mp 152-153°C).

| Elemental analysis for C₃₂H₄₆N₂O₆S | | | |
|---|---|---|---|
| Calc'd: | C, 65.50; | H, 7.90; | N, 4.77 |
| Found: | C, 65.88; | H, 8.00; | N, 4.66 |

### Example 19

### 5-[[[3,5-Bis(1,1-dimethylethyl)-4-hydroxyphenyl]amino][[[5-(2,2-dimethylpropyl)-2-thienyl]methyl](1-methylheptyl)amino]methylene]-2,2-dimethyl-1,3-dioxane-4,6-dione

This compound was synthesized using the same methodology as in Method F except 2-aminoheptane was substituted for 1-aminoheptane to yield 2.0g (85%) of a white powder (mp 184-185°C).

| Elemental analysis for C₃₈H₅₈N₂O₅S | | | |
|---|---|---|---|
| Calc'd: | C, 69.69; | H, 8.92; | N, 4.28 |
| Found: | C, 69.56; | H, 8.83; | N, 4.34 |

### Example 20

### 5-[[[Cyclohexyl-[5-(2,2-dimethylpropyl)-2-thienyl]methyl]amino]-[(2,4-dimethoxyphenyl)amino]-methylene]-2,2-dimethyl-1,3-dioxane-4,6-dione

This compound was synthesized using the same methodology as in Method E except cyclohexylamine was substituted for 1-aminoheptane to yield 1.21 g (71%) of an off-white powder (mp 143-145°C).

| Elemental analysis for C₃₁H₄₂N₂O₆S | | | |
|---|---|---|---|
| Calc'd: | C, 65.24; | H, 7.42; | N, 4.91 |
| Found: | C, 65.61; | H, 7.57; | N, 4.60 |

### Method G

### Example 21

### 5-[(Benzo[b]thiophen-3-ylmethyl-hexyl-amino)-(3,5-di-tert-butyl-4-hydroxy-phenylamino)-methylene]-2,2-dimethyl-[1,3]dioxane-4,6-dione

### Procedure 1

To a solution of 7.0 g (52.16 mmol) of thianapthene in 315 mL of anhydrous diethyl ether at -78°C was added 34 mL of 2.5 M n-butyllithium dropwise. After 0.5 hours at -78°C, the solution was warmed slowly to 0°C and a solution of 6.4 mL (52.16 mmol) of N-methylformanilide in 10 mL of diethyl ether was added dropwise. After 1.5 hours at 0°C, the reaction mixture was slowly brought to reflux for 1 hour. The mixture was cooled to room temperature and poured into 30 mL of 3N HCl. The layers were separated and the aqueous layer was extracted 3 times with 30 mL of diethyl ether. The combined ether extracts were then washed with 1N HCl (30 mL) and once with saturated aqueous NaHCO₃ (30 mL) then dried (MgSO₄) and the solvents removed at reduced pressure. The residue was dissolved in 15 mL of ethanol and 4.5 mL of saturated sodium bisulfite was added. After thorough mixing, the solution was allowed to stand at room temperature for 20 minutes. The white precipitate was filtered and washed three times with diethyl ether. After drying under vacuum, the solid was dissolved in H₂O, cooled to 0°C and saturated Na₂CO₃ was added. This aqueous layer was extracted with ethyl acetate which was dried (MgSO₄), filtered, and the solvents were removed at reduced pressure. Column chromatography of the residue on silica gel (275 g silica gel, 93% hexanes - 7% ethyl acetate to 89% hexanes - 11% ethyl acetate) gave after crystallization (diethyl ether-hexanes) 1.32 g of a solid which was used without further purification or characterization.

### Procedure 2

To a solution of 0.97 g (5.98 mmol) of 2-benzo[b]thiophenecarboxaldehyde, from Method G, Procedure 1, in 10 mL of methanol was added 1.81 g (17.9 mmol) of hexylamine and then methanol saturated with gaseous HCl until the pH was 7. Sodium cyanoborohydride, 0.25g (4.18 mmol), was added as a solid and stirred at room temperature for 18 hours. The pH of the solution was lowered to 2 with concentrated HCl and the methanol was removed at reduced pressure. H₂O was added to the residue which was washed with diethyl ether. The aqueous layer was made basic with solid KOH and the solution was extracted twice with ethyl acetate. The combined ethyl acetate layers were dried (Na₂SO₄) and the solvents removed under reduced pressure. Column chromatography of the residue (120 g silica gel, 75% EtOAc-hexanes) yielded 0.55 g (37%) of a yellow oil. NMR (200 MHz, CDCl₃) δ 7.8-7.63 (m 2H), 7.36-7.20 (m, 2H), 7.12 (s, 1H), 4.06 (s, 2H), 2.66 (t, 2H, J=6.4 Hz), 1.58-1.22 (m, 8H), 0.90 (t, 3H, J=6.6 Hz).

### Procedure 3

To a solution of 6.4 g (25.8 mmol) of 5-[bis(methylthio)methylene]-2,2-dimethyl-1,3-dioxane-4,6-dione and 4.84 g (56.0 mmol) of sodium bicarbonate in 10 mL of degassed DMSO was added 10.0 g (36.0 mmol) of 3,5-di-t-butyl-4-hydroxyaniline hydrochloride in 30 mL of degassed DMSO over a 5 hour period at room temperature. Stirring was continued for an additional 19 hours. The reaction mixture was poured into cold H₂O and the product filtered. The solid was dried and dissolved in ethyl acetate and filtered again. The solvent was removed at reduced pressure and the residue submitted to a column chromatography on silica gel (3:1 to 2:1 hexanes-ethyl acetate) to yield 9.8 g (90%) of a solid that was used without further purification.

### Procedure 4

To a solution of 0.55 g (2.22 mmol) of the amine from Method G, Procedure 2 in 20 mL CH₃CN was added 0.94 g (2.22 mmol) of the compound from Method G, Procedure 3, 0.22 g (2.22 mmol) of triethylamine and 0.36 g (1.22 mmol) of HgSO₄. The reaction mixture was allowed to reflux for 18 hours. After cooling to room temperature, the reaction mixture was filtered through Celite® and the solvents were removed at reduced pressure. Column chromatography of the residue (110 gm silica gel, 65:35 hexanes-ethyl acetate) yielded after recrystallization (ethyl acetate-hexanes) 0.95 gm (69%) of a solid (m.p. 155-159°C). IR (KBr) 3410, 3220, 2952, 2870, 1688, 1635, 1554, 1432, 1383, 1352, 1251, 1203, 1117, 1089, 929 and 740 cm⁻¹. ¹H NMR (400 MHz, CDCl₃) δ 7.76 (d, 1H, J=7.05 Hz), 7.71 (d, 1H, J=6.95 Hz), 7.35 (m, 2H), 7.24 (s, 1H), 6.95 (s, 1H), 5.23 (s, 1H), 4.69 (s, 1H), 3.09 (m, 2H), 1.69 (s, 6H), 1.63 (m, 2H), 1.35 (s, 18H), 1.26-1.19 (m, 6H), 0.83 (t, 3H, J=6.9 Hz).

| Elemental analysis for C₃₆H₄₈N₂O₅S | | | |
|---|---|---|---|
| Calc'd: | C, 69.64; | H, 7.79; | N, 4.51 |
| Found: | C, 69.81; | H, 8.09; | N, 4.38 |

### Method H

### Example 22

### 5-[[(Benzo[b]thiophen-2-ylmethyl)-hexyl-amino]-(2,4-dimethoxyphenylamino)-methylene]-2,2-dimethyl-[1,3]dioxane-4,6-

### Procedure 1

To a solution containing 2.0 g (8.05 mmol) of 5-[bis(methylthio)methylene]-2,2-dimethyl-1,3-dioxane-4,6-dione in 40 mL of t-butanol, was added 1.23 g (8.05 mmol) of 2.4-dimethoxyaniline. The reaction mixture was allowed to stir at reflux for 24 hours. The mixture was cooled to room temperature and diluted with hexanes. The solid was filtered and used without further purification. Isolated: 2.3 g, 81% yield.

### Procedure 2

To a solution of 0.76 g (3.08 mmol) of the amine from Method G, Procedure 2, in 15 mL of acetonitrile, was added 0.31 g (3.08 mmol) of triethylamine 1.08 g (3.08 mmol) of the product from Method H, Procedure 1 and 0.50 g (1.69 mmol) of HgSO₄. The reaction mixture was allowed to stir at reflux for 18 hours. The mixture was cooled to room temperature, filtered through Celite® and the solvents removed at reduced pressure. Column chromatography (120 g silica gel, 65:35 hexanes-ethyl acetate to 50:50 hexanes-ethylacetate) of the residue yielded after recrystallization (ethyl acetate-hexanes) a yellow solid (m.p. 96-99°C). IR (KBr) 3430, 2960, 2925, 2860, 1697, 1632, 1572, 1512, 1456, 1437, 1386, 1347, 1312, 1208, 1158, 1079, 1032, 930 and 788 cm⁻¹. ¹H NMR (400 MHz, CDCL₃) δ 7.79 (d, 2H, J=8.0 Hz), 7.71 (d, 1H, J=7.8 Hz), 7.33-7.28 (m, 2H), 7.25 (s, 1H), 7.14 (d, 1H, J=8.7 Hz), 6.45 (d, 1H, J=2.7 Hz), 6.30 (dd, 1H, J=2.68, 8.72 Hz), 4.70 (s, 2H), 3.75 (s, 6H), 3.07 (m, 2H), 1.64-1.56 (m, 8H), 1.22-1.12 (m, 6H), 0.80 (t, 3H, J=6.84 Hz).

| Elemental analysis for C₃₀H₃₆N₂O₆S | | | |
|---|---|---|---|
| Calc'd: | C, 65.20; | H, 6.57; | N, 5.07 |
| Found: | C, 64.85; | H, 6.58; | N, 5.02 |

### Example 23

### 5-[[Benzo[b]thiophen-2-ylmethyl-(1-methyl-hexyl)-amino]-(2,4-dimethoxy-phenylamino)-methylene]-2,2-dimethyl-[1,3]dioxane-4,6-dione

This compound was synthesized using the methodology described in Method H except 2-aminoheptane was substituted for hexylamine to yield a white solid (m.p. 167°C).

| Elemental analysis for C₃₁H₃₈N₂O₆S | | | |
|---|---|---|---|
| Calc'd: | C, 65.70; | H, 6.76; | N, 4.94 |
| Found: | C, 65.64; | H, 6.67; | N, 4.84 |

### Example 24

### 5-[[Benzo[b]thiophen-2-ylmethyl-(1-methyl-hexyl)-amino]-(3,5-di-tert-butyl-4-hydroxy-phenylamino)methylene]-2,2-dimethyl-[1,3]dioxane-4,6-dione

This compound was synthesized using the methodology described in Method G except 2-aminoheptane was substituted for hexylamine to yield a white solid (m.p. 195-197°C).

| Elemental analysis for C₃₇H₅₀N₂O₅S | | | |
|---|---|---|---|
| Calc'd: | C, 70.00; | H, 7.94; | N, 4.41 |
| Found: | C, 70.21; | H, 7.85; | N, 4.46 |

The ability of the compounds of this invention to inhibit acyl-coenzyme A: cholesterol acyltransferase was established by initially showing that they inhibited intracellular cholesterol esterification by subjecting them to the standard experimental test procedure of Ross et al., J. Biol. Chem. 259 815 (1984). The results of these studies are presented in:

**Table I**

| Example | % Inhib, (Concentration - µM) | IC₅₀(µM) |
|---|---|---|
| 1 | 69 (25) | 2.96 |
| 3 | 55 (25) | 2.33 |
| 4 | 72 (25) | 5.16 |
| 5 | 90 (25) | 2.42 |
| 6 | 95 (25) | 1.55 |
| 7 | 51 (25) | 9.41 |
| 8 | 97 (25) | 1.65 |
| 9 | 98 (25) | 1.35 |
| 10 | 94 (25) | 5.82 |
| 11 | 24 (25) | - |
| 12 | 92 (25) | 4.11 |
| 13 | 26 (25) | - |
| 14 | 18 (25) | - |
| 15 | 97 (25) | 3.9 |
| 16 | 94 (25) | 2.96 |
| 17 | - | 0.70 |
| 18 | 97 (25) | - |
| 19 | - | 0.64 |
| 20 | 95 (25) | - |
| 21 | 98 (25) | 0.38 |
| 22 | 94 (25) | 0.62 |
| 23 | 97 (25) | 0.35 |
| 24 | 98 (25) | 0.31 |

Representative compounds were further tested in vivo to establish the percent inhibition of cholesterol absorption. In this study, normal rats were dosed (oral gavage) with ¹⁴C-cholesterol plus the test compound. Blood samples taken exactly six hours later were analyzed and the percent inhibition of cholesterol absorption was calculated as shown in:

**Table II**

| In Vivo Testing ¹⁴C-Cholesterol Absorption in Normal Rats | | |
|---|---|---|
| Example | Dose mg/kg | % Inhibition of Absorption |
| 1 | 10 | 72 |
| 2 | 3 | 2 |
| 3 | 3 | 11 |
| 12 | 10 | 65 |
| 15 | 3 | 48 |

In addition, the product of Example 1 was studied in vivo in the cholesterol-cholic acid fed rat to determine the percent decrease of cholesterol in their plasma This study involves rats which are, prior to testing, trained for one week to eat over a four hour time period each day. Upon initiation of the experiment, the rats diet is supplemented with 1.0 percent cholesterol and 0.25 percent cholic acid. The rats are dosed with the test compound by oral gavage just prior to and just following the four hour feeding period. This is repeated for four days. On the fifth day, the rats are sacrificed and the total plasma cholesterol content is determined. The percent decrease in elevated plasma cholesterol levels is calculated in comparison with normal-fed controls. The compound of Example 1 at 10 mg/kg resulted in a 50 percent decrease in plasma cholesterol.

From these data, the ability of the compounds to inhibit ACAT is clearly established. Hence, the compounds of this invention are useful in the treatment of those disease states which are amenable to treatment by reduction of the rate of cholesterol esterification, the rate of accumulation and deposits of cholesteryl esters on arterial walls and the rate of formation of atheromatous lesions. As such, the anti-atherosclerotic agents of this invention may be administered to a mammal in need of intracellular cholesteryl ester concentration reduction orally or parenterally in an amount sufficient to inhibit ACAT catalysis of cholesterol esterification.

The compounds of this invention may be administered by themselves or in combination with pharmaceutically acceptable liquid or solid carriers. Oral administration in conventional formulations as tablets, capsules, powders, or suspensions is preferred.

A solid carrier can include one or more substances which may also act as flavoring agents, lubricants, solubilisers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both of pharmaceutically acceptable oils or fats. The liquid carrier can contain other suitable pharmaceutical additives such as solubilisers, emulsifiers, buffers, preservatives, sweeteners, flavoring agents, suspending agents, thickening agents, colors, viscosity regulators, stabilisers or osmo-regulators. Suitable examples of liquid carriers for oral and parenteral administration include water (particularly containing additives as above, e.g. cellulose derivatives, preferably sodium carboxymethyl cellulose solution), alcohols (including monohydric alcohols and polyhydric alcohols, e.g., glycols) and their derivatives, and oils (e.g. fractionated coconut oil and arachis oil). For parenteral administration, the carrier can also be an oil ester such as ethyl oleate and isopropyl myristate. Sterile liquid carriers are used in sterile liquid form compositions for parenteral administration. The liquid carrier for pressurized compositions can be halogenated hydrocarbon or other pharmaceutically acceptable propellent.

Liquid pharmaceutical compositions which are sterile solutions or suspensions can be utilized by, for example, intramuscular intraperitoneal or subcutaneous injection. Sterile solutions can also be administered intravenously. When the compound is orally active, it can be administered orally either in liquid or solid composition form.

Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

The dosage to be used in the treatment of a specific hypercholesterolemic/atherosclerotic condition must be subjectively determined by the attending physician. The variables involved include the extent of the disease state, size, age and response pattern of the patient.

## Claims (Claims for the following Contracting State(s): AT, BE, CH, DE, DK, FR, GB, IT, LI, LU, NL, SE)

1. A compound of the formula: 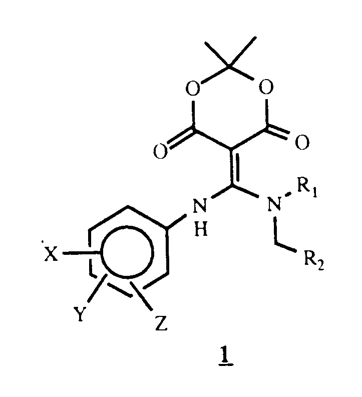 in which
X, Y and Z are, independently, hydrogen, halogen, hydroxy, nitro, cyano, carboxyl, trifluoromethyl, phenyl, amino, alkylamino of 1 to 12 carbon atoms, dialkylamino in which each alkyl group has 1 to 12 carbon atoms, alkyl of 1 to 12 carbon atoms or alkoxy of 1 to 12 carbon atoms;
R₁ is hydrogen, alkyl of 1 to 18 carbon atoms, alkenyl of 2 to 18 carbon atoms, cycloalkyl of 5 to 8 carbon atoms, phenyl, benzyl or substituted phenyl or benzyl where the substituents are alkyl of 1 to 12 carbon atoms or alkoxy of 1 to 12 carbon atoms;
R₂ is 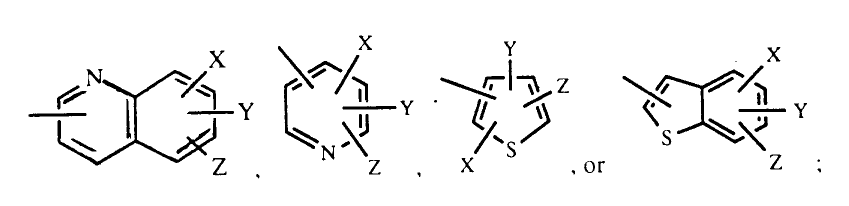 in which X, Y and Z independently have a value as defined above for X, Y and Z, the same or different, or a pharmaceutically acceptable salt thereof.

2. A compound of Claim 1 of the formula: 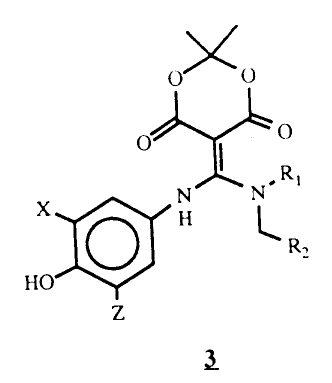 in which
X and Z are, independently, alpha branched alkyl of 1 to 6 carbon atoms;
R₁ is alkyl of 1 to 18 carbon atoms;
and
R₂ is 2-, 3- or 4-quinolinyl; or a pharmaceutically acceptable salt thereof.

3. A compound of Claim 1 of the formula: 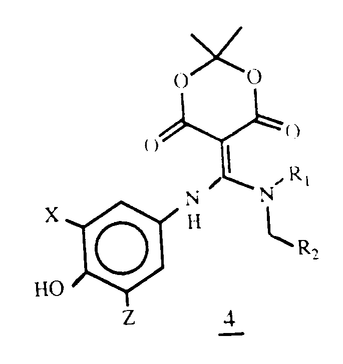 in which
X and Z are, independently, alpha branched alkyl of 1 to 6 carbon atoms;
R₁ is alkyl of 1 to 18 carbon atoms;
and
R₂ is 2-, 3- or 4- pyridinyl; or a pharmaceutically acceptable salt thereof.

4. A compound of Claim 1 of the formula: 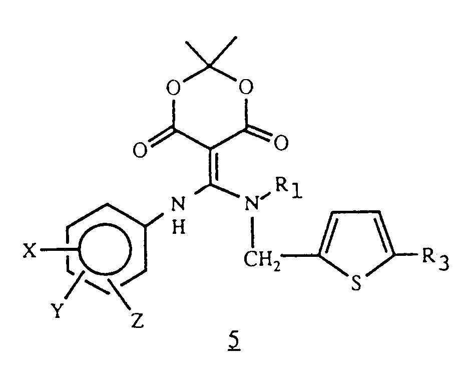 in which
X, Y and Z are, independently, hydrogen, alkyl of 1 to 6 carbon atoms, alkoxy of 1 to 6 carbon atoms, or hydroxy;
R₁ is alkyl of 6 to 10 carbon atoms or cycloalkyl of 5 to 7 carbon atoms; and
R₃ is alkyl of 1 to 6 carbon atoms.

5. A compound of Claim 1 of the formula: 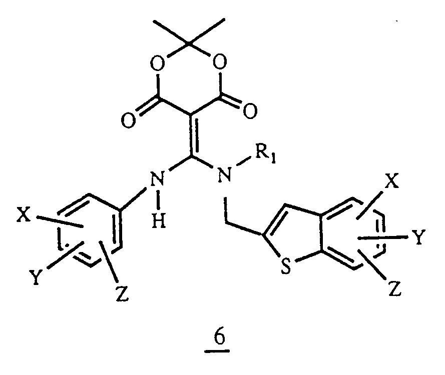 in which
X, Y and Z are, independently, alkyl of 1 to 4 carbon atoms, alkoxy of 1 to 4 carbon atoms or hydroxy; and
R₁ is alkyl of 1 to 18 carbon atoms; or a pharmaceutically acceptable salt thereof.

6. A compound of Claim 1 which is 5-[[[3,5-bis(1,1-dimethylethyl)-4-hydroxyphenyl]amino][hexyl(4-quinolinylmethyl)amino]methylene]-2,2-dimethyl-1,3-dioxane-4,6-dione, or a pharmaceutically acceptable salt thereof.

7. A compound of Claim 1 which is 5-[[[3,5-bis(1,1-dimethylethyl)-4-hydroxyphenyl]amino][hexyl(3-quinolinylmethyl)amino]methylene-2,2-dimethyl-1,3-dioxane-4,6-dione, or a pharmaceutically acceptable salt thereof.

8. A compound of Claim 1 which is 5-[(3,5-di-tert-butyl-4-hydroxyphenylamino)-(hexyl-(pyridin-4-ylmethyl)-amino)-methylene]-2,2-dimethyl-1,3-dioxane-4,6-dione, or a pharmaceutically acceptable salt thereof.

9. A compound of Claim 1 which is 5-[[[3,5-bis(1,1-dimethylethyl)-4-hydroxyphenyl]amino][[[5-(2,2-dimethylpropyl)-2-thienyl]methyl]heptylamino]methylene]-2,2-dimethyl-1,3-dioxane-4,6-dione.

10. A compound of Claim 1 which is 5-[[[3,5-bis(1,1-dimethylethyl)-4-hydroxyphenyl]amino][[[5-2,2-dimethylpropyl)-2-thienyl]methyl](1-methylheptyl)amino]methylene]-2,2-dimethyl-1,3-dioxane-4,6-dione.

11. A compound of Claim 1 which is 5-[[benzo[b]thiophen-2-ylmethyl-(1-methyl-hexyl)amino]-(3,5-di-tert-butyl-4-hydroxy-phenylamino)methylene]-2,2-dimethyl-[1,3]dioxan-4,5-dione, or a pharmaceutically acceptable salt thereof.

12. A process for preparing a compound of formula **1** as defined in Claim 1 or a pharmaceutically acceptable salt thereof which comprises
a) reacting a compound of formula 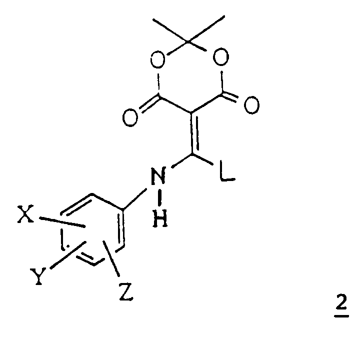 wherein X, Y and Z are as defined above and L is a leaving group with an amine of formula HN(R₁)CH₂R₂ where
R₁ and R₂ are as defined in Claim 1,
or
b) reacting 2,2-dimethyl-1,3-dioxane-4,6-dione (Meldrum's Acid) with a carbodiimide of formula 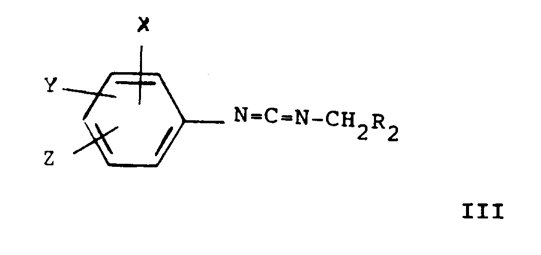 where R₂ is as defined in Claim 1 to give a compound of formula I as defined above wherein R₁ is hydrogen, and if desired after said process a) or b) converting any value of X, Y or Z to one of the other values for X, Y and Z by known methods and further if desired isolating the product as a pharmaceutically acceptable salt.

13. A pharmaceutical composition comprising a compound of formula I or a pharmaceutically acceptable salt thereof as defined in any one of Claims 1 to 11 and a pharmaceutically acceptable carrier.

## Claims (Claims for the following Contracting State(s): ES, GR)

1. A process for preparing a compound of the formula: 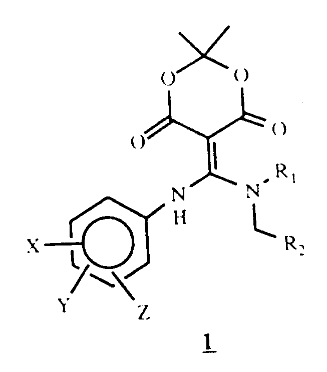 in which
X, Y and Z are, independently, hydrogen, halogen, hydroxy, nitro, cyano, carboxyl, trifluoromethyl, phenyl, amino, alkylamino of 1 to 12 carbon atoms, dialkylamino in which each alkyl group has 1 to 12 carbon atoms, alkyl of 1 to 12 carbon atoms or alkoxy of 1 to 12 carbon atoms;
R₁ is hydrogen, alkyl of 1 to 18 carbon atoms, alkenyl of 2 to 18 carbon atoms, cycloalkyl of 5 to 8 carbon atoms, phenyl, benzyl or substituted phenyl or benzyl where the substituents are alkyl of 1 to 12 carbon atoms or alkoxy of 1 to 12 carbon atoms;
R₂ is 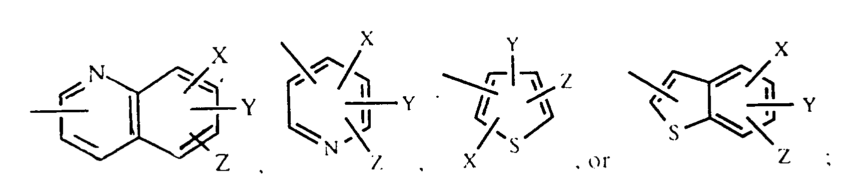 in which X, Y and Z independently have a value as defined above for X, Y and Z, the same or different, or a pharmaceutically acceptable salt thereof, which comprises
a) reacting a compound of formula 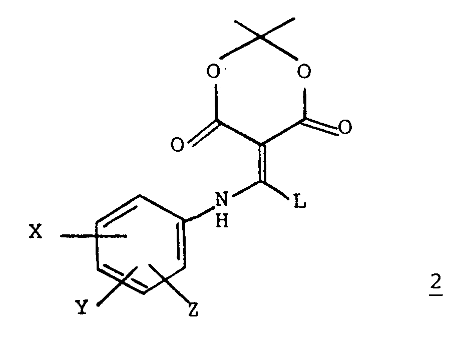 wherein X, Y and Z are as defined above and L is a leaving group with an amine of formula HN(R₁)CH₂R₂ where
R₁ and R₂ are as defined above,
or
b) reacting 2,2-dimethyl-1,3-dioxane-4,6-dione (Meldrum's Acid) with a carbodiimide of formula 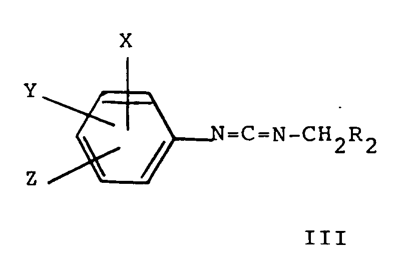 where R₂ is as defined above to give a compound of formula I as defined above wherein R₁ is hydrogen; and if desired after said process a) or b) converting any value of X, Y or Z to one of the other values for X, Y and Z by known methods and further if desired isolating the product as a pharmaceutically acceptable salt.

2. A process of Claim 1 in which the compound prepared has the formula: 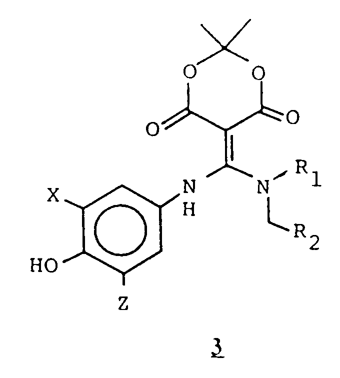 in which
X and Z are, independently, alpha branched alkyl of 1 to 6 carbon atoms;
R₁ is alkyl of 1 to 18 carbon atoms; and
R₂ is 2-, 3- or 4-quinolinyl;
or a pharmaceutically acceptable salt thereof.

3. A process of Claim 1 in which the compound prepared has the formula: 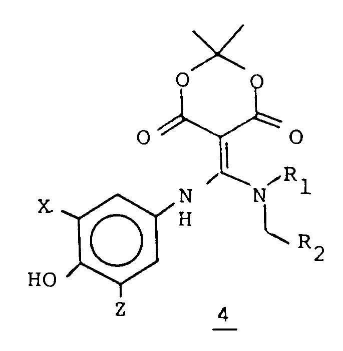 in which
X and Z are, independently, alpha branched alkyl of 1 to 6 carbon atoms;
R₁ is alkyl of 1 to 18 carbon atoms;
and
R₂ is 2-, 3- or 4- pyridinyl; or a pharmaceutically acceptable salt thereof.

4. A process of Claim 1 in which the compound prepared has the formula: 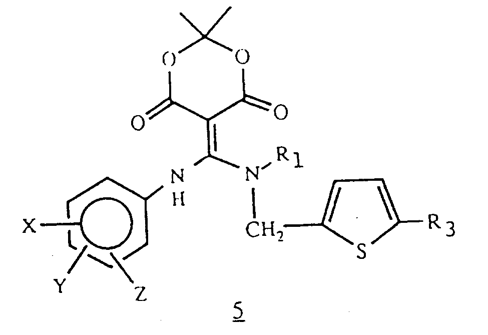 in which
X, Y and Z are, independently, hydrogen, alkyl of 1 to 6 carbon atoms, alkoxy of 1 to 6 carbon atoms, or hydroxy;
R₁ is alkyl of 6 to 10 carbon atoms or cycloalkyl of 5 to 7 carbon atoms; and
R₃ is alkyl of 1 to 6 carbon atoms.

5. A process of Claim 1 in which the compound prepared has the formula: 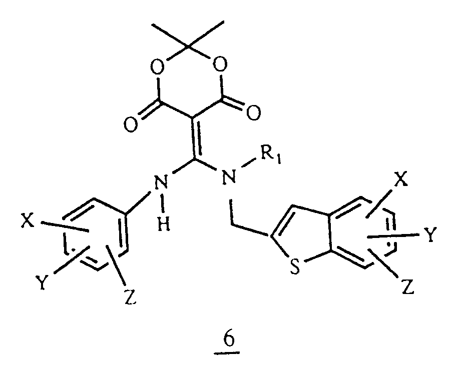 in which
X, Y and Z are, independently, alkyl of 1 to 4 carbon atoms, alkoxy of 1 to 4 carbon atoms or hydroxy; and
R₁ is alkyl of 1 to 18 carbon atoms; or a pharmaceutically acceptable salt thereof.

6. A process of Claim 1 in which the compound prepared is 5-[[[3,5-bis(1,1-dimethylethyl)-4-hydroxyphenyl]amino][hexyl(4-quinolinylmethyl)amino]methylene]-2,2-dimethyl-1,3-dioxane-4,6-dione, or a pharmaceutically acceptable salt thereof.

7. A process of Claim 1 in which the compound prepared is 5-[[[3,5-bis(1,1-dimethylethyl)-4-hydroxyphenyl]amino][hexyl(3-quinolinylmethyl)amino]methylene-2,2-dimethyl-1,3-dioxane-4,6-dione, or a pharmaceutically acceptable salt thereof.

8. A process of Claim 1 in which the compound prepared is 5-[(3,5-di-tert-butyl-4-hydroxyphenylamino)-(hexyl-(pyridin-4-ylmethyl)-amino)-methylene]-2,2-dimethyl-1,3-dioxane-4,6-dione, or a pharmaceutically acceptable salt thereof.

9. A process of Claim 1 in which the compound prepared is 5-[[[3,5-bis(1,1-dimethylethyl)-4-hydroxyphenyl]amino][[[5-(2,2-dimethylpropyl)-2-thienyl]methyl]heptylamino]-methylene]-2,2-dimethyl-1,3-dioxane-4,6-dione.

10. A process of Claim 1 in which the compound prepared is 5-[[[3,5-bis(1,1-dimethylethyl)-4-hydroxyphenyl]amino][[[5-2,2-dimethylpropyl)-2-thienyl]methyl](1-methylheptyl)-amino]methylene]-2,2-dimethyl-1,3-dioxane-4,6-dione.

11. A process of Claim 1 in which the compound prepared is 5-[[benzo[b]thiophen-2-ylmethyl-(1-methylhexyl)-amino]-(3,5-di-tert-butyl-4-hydroxy-phenylamino)-methylene]-2,2-dimethyl-[1,3]dioxane-4,6-dione, or a pharmaceutically acceptable salt thereof.

12. A process for preparing a pharmaceutical composition which comprises bringing a compound of formula I or a pharmaceutically acceptable salt thereof as defined in any one of Claims 1 to 11 into association with a pharmaceutically acceptable carrier.

## Patentansprüche (Patentansprüche für folgende(n) Vertragsstaat(en): AT, BE, CH, DE, DK, FR, GB, IT, LI, LU, NL, SE)

1. Verbindung der Formel: 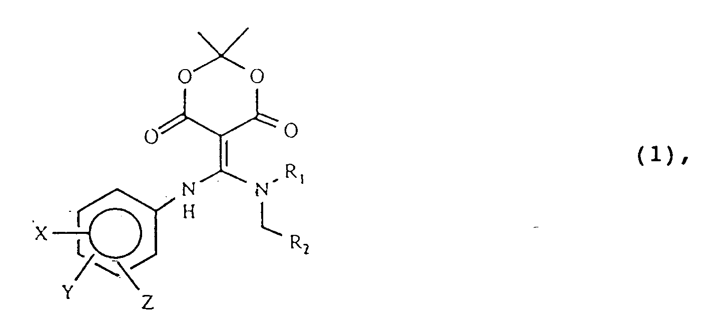 worin X, Y und Z unabhängig Wasserstoff, Halogen, Hydroxy, Nitro, Cyano, Carboxyl, Trifluormethyl, Phenyl, Amino, Alkylamino mit 1 bis 12 Kohlenstoffatomen, Dialkylamino, wobei jede Alkyl-Gruppe 1 bis 12 Kohlenstoffatome aufweist, Alkyl mit 1 bis 12 Kohlenstoffatomen oder Alkoxy mit 1 bis 12 Kohlenstoffatomen bedeuten; R₁ Wasserstoff, Alkyl mit 1 bis 18 Kohlenstoffatomen, Alkenyl mit 2 bis 18 Kohlenstoffatomen, Cycloalkyl mit 5 bis 8 Kohlenstoffatomen, Phenyl, Benzyl oder substituiertes Phenyl oder Benzyl, wobei die Substituenten Alkyl mit 1 bis 12 Kohlenstoffatomen oder Alkoxy mit 1 bis 12 Kohlenstoffatomen sind, darstellt; R₂ bedeutet: 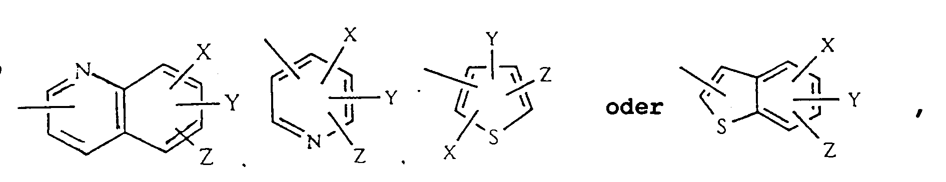 wobei X, Y und Z unabhängig eine Bedeutung haben, wie oben für X, Y und Z definiert, die gleich oder verschieden sind, oder ein pharmazeutisch annehmbares Salz hievon.

2. Verbindung nach Anspruch 1 der Formel: 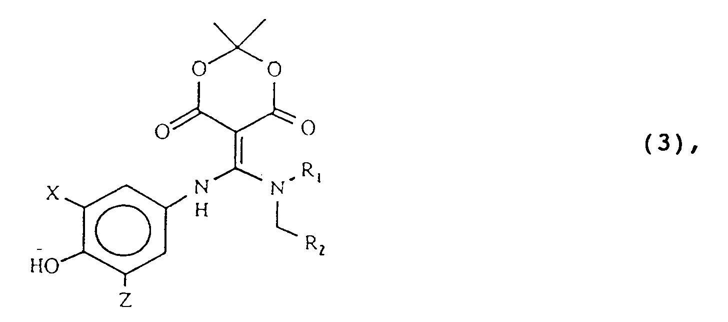 worin X und Z unabhängig alpha-verzweigtes Alkyl mit 1 bis 6 Kohlenstoffatomen bedeuten; R₁ Alkyl mit 1 bis 18 Kohlenstoffatomen darstellt; und R₂ 2-, 3- oder 4-Chinolinyl ist; oder ein pharmazeutisch annehmbares Salz hievon.

3. Verbindung nach Anspruch 1 der Formel: 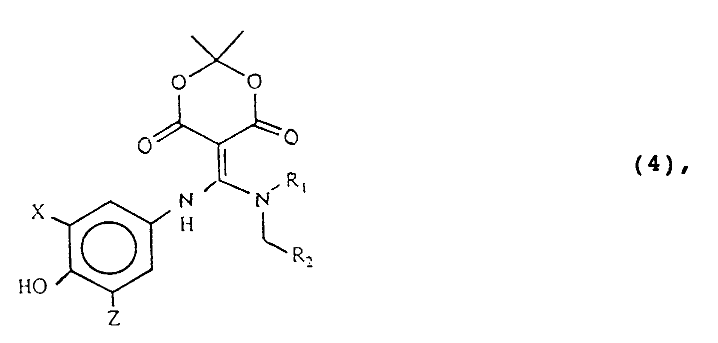 worin X und Z unabhängig alpha-verzweigtes Alkyl mit 1 bis 6 Kohlenstoffatomen bedeuten; R₁ Alkyl mit 1 bis 18 Kohlenstoffatomen darstellt; und R₂ 2-, 3- oder 4-Pyridinyl ist; oder ein pharmazeutisch annehmbares Salz hievon.

4. Verbindung nach Anspruch 1 der Formel: 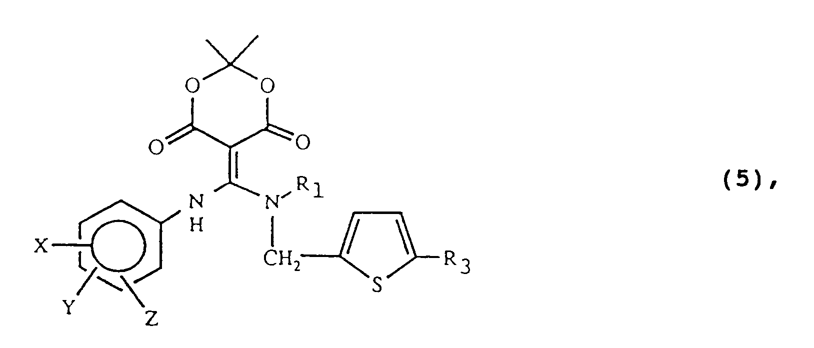 worin X, Y und Z unabhängig Wasserstoff, Alkyl mit 1 bis 6 Kohlenstoffatomen, Alkoxy mit 1 bis 6 Kohlenstoffatomen oder Hydroxy bedeuten; R₁ Alkyl mit 6 bis 10 Kohlenstoffatomen oder Cycloalkyl mit 5 bis 7 Kohlenstoffen darstellt; und R₃ Alkyl mit 1 bis 6 Kohlenstoffatomen ist.

5. Verbindung nach Anspruch 1 der Formel: 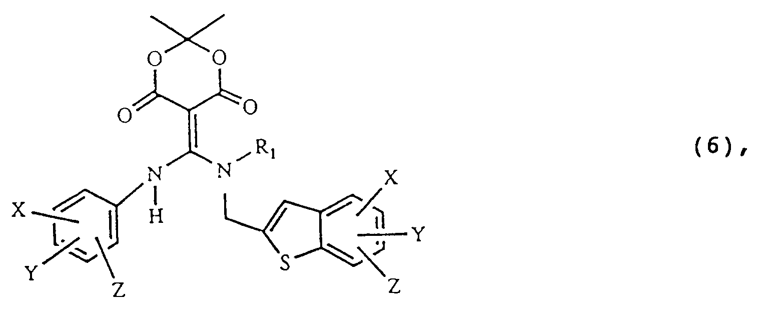 worin X, Y und Z unabhängig Alkyl mit 1 bis 4 Kohlenstoffatomen, Alkoxy mit 1 bis 4 Kohlenstoffatomen oder Hydroxy bedeuten; und R₁ Alkyl mit 1 bis 18 Kohlenstoffatomen darstellt; oder ein pharmazeutisch annehmbares Salz hievon.

6. Verbindung nach Anspruch 1, nämlich 5-[[[3,5-Bis-(1,1-dimethylethyl)-4-hydroxyphenyl]-amino]-[hexyl-(4-chinolinylmethyl)-amino]-methylen]-2,2-dimethyl-1,3-dioxan-4,6-dion, oder ein pharmazeutisch annehmbares Salz hievon.

7. Verbindung nach Anspruch 1, nämlich 5-[[[3,5-Bis-(1,1-dimethylethyl)-4-hydroxyphenyl]-amino]-[hexyl-(3-chinolinylmethyl)-amino]-methylen]-2,2-dimethyl-1,3-dioxan-4,6-dion, oder ein pharmazeutisch annehmbares Salz hievon.

8. Verbindung nach Anspruch 1, nämlich 5-[(3,5-Di-tert.butyl-4-hydroxyphenylamino)-(hexyl-(pyridin-4-ylmethyl)-amino)-methylen]-2,2-dimethyl-1,3-dioxan-4,6-dion, oder ein pharmazeutisch annehmbares Salz hievon.

9. Verbindung nach Anspruch 1, nämlich 5-[[[3,5-Bis-(1,1-dimethylethyl)-4-hydroxyphenyl]-amino]-[[[5-(2,2-dimethylpropyl)-2-thienyl]-methyl]-heptylamino]-methylen]-2,2-dimethyl-1,3-dioxan-4,6-dion.

10. Verbindung nach Anspruch 1, nämlich 5-[[[3,5-Bis-(1,1-dimethylethyl)-4-hydroxyphenyl]-amino]-[[[5-(2,2-dimethylpropyl)-2-thienyl]-methyl]-(1-methylheptyl)-amino]-methylen]-2,2-dimethyl-1,3-dioxan-4,6-dion.

11. Verbindung nach Anspruch 1, nämlich 5-[[Benzo[b]thiophen-2-ylmethyl)-(1-methylhexyl)-amino]-(3,5-di-tert.butyl-4-hydroxyphenylamino)-methylen]-2,2-dimethyl[1,3]dioxan-4,6-dion oder ein pharmazeutisch annehmbares Salz hievon.

12. Verfahren zur Herstellung einer Verbindung der Formel (I), wie in Anspruch 1 definiert, oder eines pharmazeutisch annehmbaren Salzes hievon, welches umfaßt:
a) Umsetzen einer Verbindung der Formel 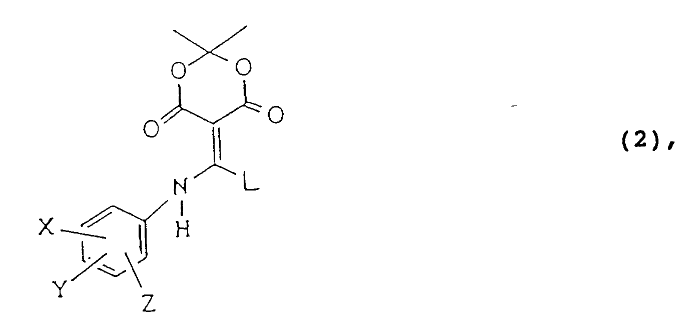 worin X, Y und Z wie oben definiert sind, und L eine Abgangsgruppe bedeutet, mit einem Amin der Formel HN(R₁)CH₂R₂, wobei R₁ und R₂ wie in Anspruch 1 definiert sind, oder
b) Umsetzen von 2,2-Dimethyl-1,3-dioxan-4,6-dion (Meldrumsäure) mit einem Carbodiimid der Formel 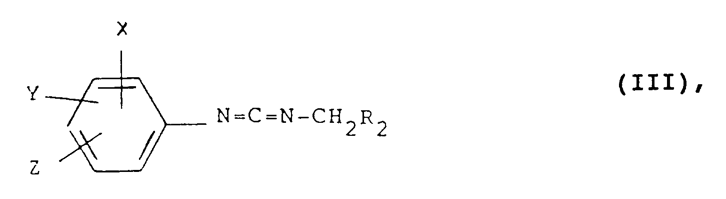 worin R₂ wie in Anspruch 1 definiert ist, wobei eine Verbindung der Formel (1), wie oben definiert, wobei R₁ Wasserstoff bedeutet, erhalten wird; und, wenn gewüscht, nach dem genannten Verfahren a) oder b) Überführen jeder Bedeutung von X, Y oder Z in eine der anderen Bedeutungen für X, Y und Z durch bekannte Verfahren, und weitere, wenn gewünscht, Isolieren des Produkts als pharmazeutisch annehmbares Salz.

13. Pharmazeutische Zusammensetzung, welche eine Verbindung der Formel (1) oder ein pharmazeutisch annehmbares Salz hievon, wie in einem der Ansprüche 1 bis 11 definiert, und einen pharmazeutisch annehmbaren Träger umfaßt.

## Patentansprüche (Patentansprüche für folgende(n) Vertragsstaat(en): ES, GR)

1. Verfahren zur Herstellung einer Verbindung der Formel: 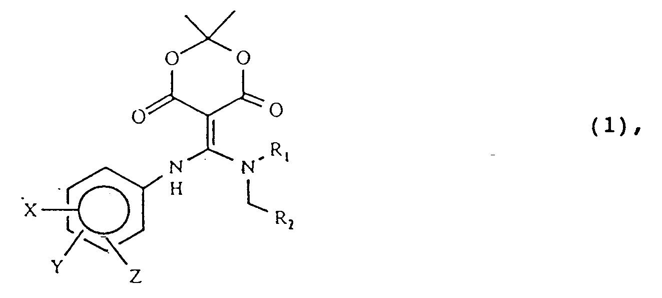 worin X, Y und Z unabhängig Wasserstoff, Halogen, Hydroxy, Nitro, Cyano, Carboxyl, Trifluormethyl, Phenyl, Amino, Alkylamino mit 1 bis 12 Kohlenstoffatomen, Dialkylamino, wobei jede Alkyl-Gruppe 1 bis 12 Kohlenstoffatome aufweist, Alkyl mit 1 bis 12 Kohlenstoffatomen oder Alkoxy mit 1 bis 12 Kohlenstoffatomen bedeuten; R₁ Wasserstoff, Alkyl mit 1 bis 18 Kohlenstoffatomen, Alkenyl mit 2 bis 18 Kohlenstoffatomen, Cycloalkyl mit 5 bis 8 Kohlenstoffatomen, Phenyl, Benzyl oder substituiertes Phenyl oder Benzyl, wobei die Substituenten Alkyl mit 1 bis 12 Kohlenstoffatomen oder Alkoxy mit 1 bis 12 Kohlenstoffatomen sind, darstellt; R₂ bedeutet: 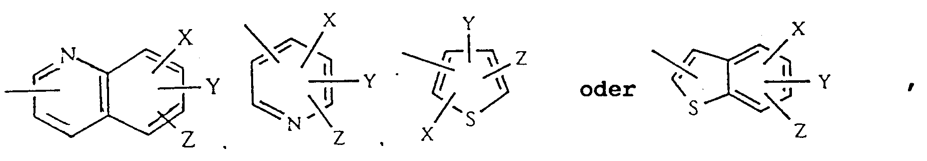 wobei X, Y und Z unabhängig eine Bedeutung haben, wie oben für X, Y und Z definiert, die gleich oder verschieden sind, oder eines pharmazeutisch annehmbaren Salzes hievon, welches Verfahren umfaßt:
a) Umsetzen einer Verbindung der Formel 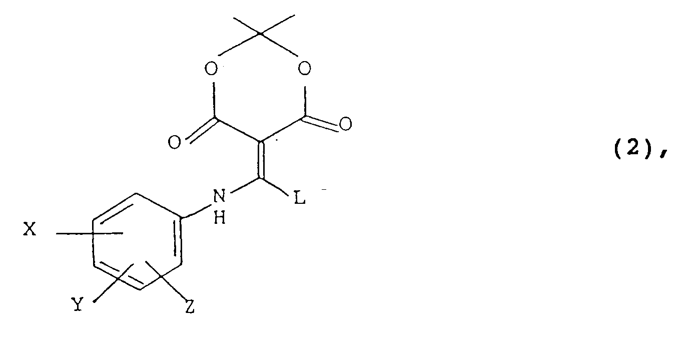 worin X, Y und Z wie oben definiert sind, und L eine Abgangsgruppe bedeutet, mit einem Amin der Formel HN(R₁)CH₂R₂, wobei R₁ und R₂ wie oben definiert sind, oder
b) Umsetzen von 2,2-Dimethyl-1,3-dioxan-4,6-dion (Meldrumsäure) mit einem Carbodiimid der Formel 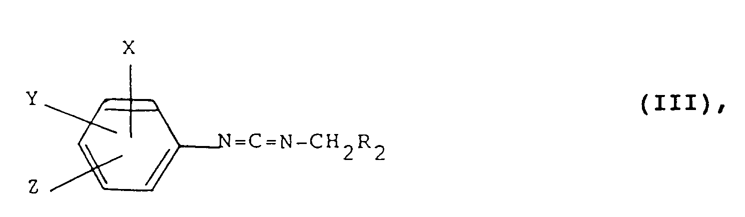 worin R₂ wie oben definiert ist, wobei eine Verbindung der Formel (1), wie oben definiert, wobei R₁ Wasserstoff bedeutet, erhalten wird; und, wenn gewüscht, nach dem genannten Verfahren a) oder b) Überführen jeder Bedeutung von X, Y oder Z in eine der anderen Bedeutungen für X, Y und Z durch bekannte Verfahren, und weiters, wenn gewünscht, Isolieren des Produkts als pharmazeutisch annehmbares Salz.

2. Verfahren nach Anspruch 1, bei welchem die hergestellte Verbindung die Formel aufweist: 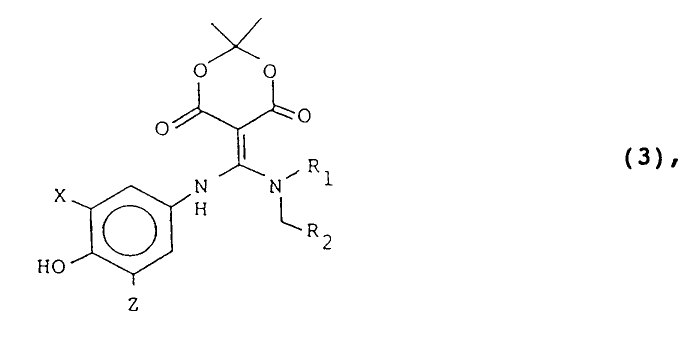 worin X und Z unabhängig alpha-verzweigtes Alkyl mit 1 bis 6 Kohlenstoffatomen bedeuten; R₁ Alkyl mit 1 bis 18 Kohlenstoffatomen darstellt; und R₂ 2-, 3- oder 4-Chinolinyl ist; oder ein pharmazeutisch annehmbares Salz hievon ist.

3. Verfahren nach Anspruch 1, bei welchem die hergestellte Verbindung die Formel aufweist: 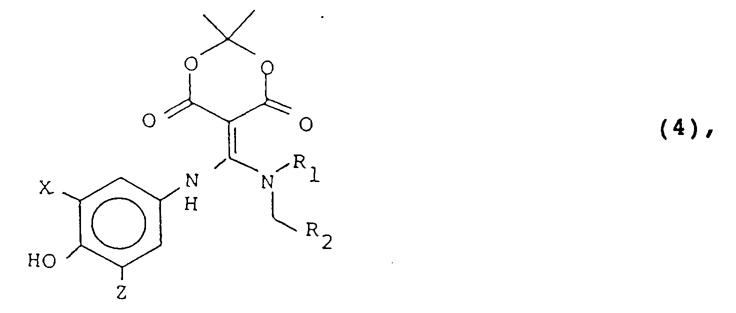 worin X und Z unabhängig alpha-verzweigtes Alkyl mit 1 bis 6 Kohlenstoffatomen bedeuten; R₁ Alkyl mit 1 bis 18 Kohlenstoffatomen darstellt; und R₂ 2-, 3- oder 4-Pyridinyl ist; oder ein pharmazeutisch annehmbares Salz hievon ist.

4. Verfahren nach Anspruch 1, bei welchem die hergestellte Verbindung die Formel aufweist: 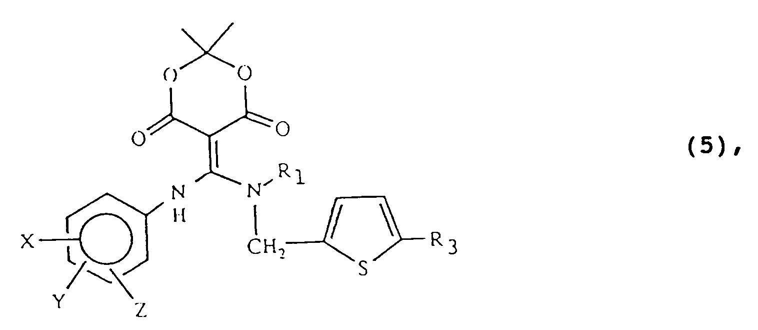 worin X, Y und Z unabhängig Wasserstoff, Alkyl mit 1 bis 6 Kohlenstoffatomen, Alkoxy mit 1 bis 6 Kohlenstoffatomen oder Hydroxy bedeuten; R₁ Alkyl mit 6 bis 10 Kohlenstoffatomen oder Cycloalkyl mit 5 bis 7 Kohlenstoffen darstellt; und R₃ Alkyl mit 1 bis 6 Kohlenstoffatomen ist.

5. Verfahren nach Anspruch 1, bei welchem die hergestellte Verbindung die Formel aufweist: 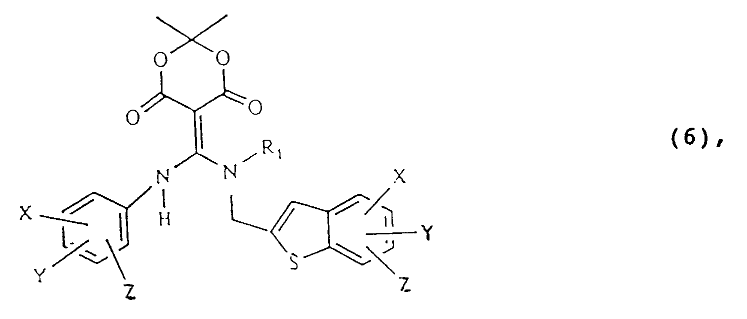 worin X, Y und Z unabhängig Alkyl mit 1 bis 4 Kohlenstoffatomen, Alkoxy mit 1 bis 4 Kohlenstoffatomen oder Hydroxy bedeuten; und R₁ Alkyl mit 1 bis 18 Kohlenstoffatomen darstellt; oder ein pharmazeutisch annehmbares Salz hievon ist.

6. Verfahren nach Anspruch 1, bei welchem die hergestellte Verbindung 5-[[[3,5-Bis-(1,1-dimethylethyl)-4-hydroxyphenyl]-amino]-[hexyl-(4-chinolinylmethyl)-amino]-methylen]-2,2-dimethyl-1,3-dioxan-4,6-dion oder ein pharmazeutisch annehmbares Salz hievon ist.

7. Verfahren nach Anspruch 1, bei welchem die hergestellte Verbindung 5-[[[3,5-Bis-(1,1-dimethylethyl)-4-hydroxyphenyl]-amino]-[hexyl-(3-chinolinylmethyl)-amino]-methylen]-2,2-dimethyl-1,3-dioxan-4,6-dion oder ein pharmazeutisch annehmbares Salz hievon ist.

8. Verfahren nach Anspruch 1, bei welchem die hergestellte Verbindung 5-[(3,5-Di-tert.butyl-4-hydroxyphenylamino)-(hexyl-(pyridin-4-ylmethyl)-amino)-methylen]-2,2-dimethyl-1,3-dioxan-4,6-dion oder ein pharmazeutisch annehmbares Salz hievon ist.

9. Verfahren nach Anspruch 1, bei welchem die hergestellte Verbindung 5-[[[3,5-Bis-(1,1-dimethylethyl)-4-hydroxyphenyl]-amino]-[[[5-(2,2-dimethylpropyl)-2-thienyl]-methyl]-heptylamino]-methylen]-2,2-dimethyl-1,3-dioxan-4,6-dion ist.

10. Verfahren nach Anspruch 1, bei welchem die hergestellte Verbindung 5-[[[3,5-Bis-(1,1-dimethylethyl)-4-hydroxyphenyl]-amino]-[[[5-(2,2-dimethylpropyl)-2-thienyl]-methyl]-(1-methylheptyl)-amino]-methylen]-2,2-dimethyl-1,3-dioxan-4,6-dion ist.

11. Verfahren nach Anspruch 1, bei welchem die hergestellte Verbindung 5-[[Benzo[b]thiophen-2-ylmethyl)-(1-methylhexyl)-amino]-(3,5-di-tert.butyl-4-hydroxyphenylamino)-methylen]-2,2-dimethyl[1,3]dioxan-4,6-dion oder ein pharmazeutisch annehmbares Salz hievon ist.

12. Verfahren zur Herstellung einer pharmazeutischen ZusammenSetzung, welches das Vereinigen einer Verbindung der Formel (1) oder eines pharmazeutisch annehmbaren Salzes hievon, wie in einem der Ansprüche 1 bis 11 definiert, mit einem pharmazeutisch annehmbaren Träger umfaßt.

## Revendications (Revendications pour l'(les) Etat(s) contractant(s) suivant(s): AT, BE, CH, DE, DK, FR, GB, IT, LI, LU, NL, SE)

1. Composé de formule : 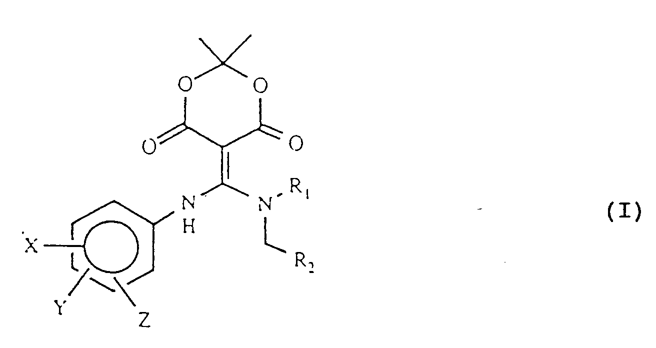 dans lequel :
X, Y et Z sont, indépendamment, hydrogène, halogène, hydroxyle, nitro, cyano, carboxyle, trifluorométhyle, phényle, amino, alcoylamino en 1 à 12 atomes de carbone, dialcoylamino dans lequel chaque radical alcoyle comprend de 1 à 12 atomes de carbone, alcoyle en 1 à 12 atomes de carbone ou alcoxy en 1 à 12 atomes de carbone;
R₁ est hydrogène, alcoyle en 1 à 18 atomes de carbone, alcényle en 2 à 18 atomes de carbone, cycloalcoyle en 5 à 8 atomes de carbone, phényle, benzyle ou phényle ou benzyle substitué, où les substituants sont alcoyle en 1 à 12 atomes de carbone ou alcoxy en 1 à 12 atomes de carbone;
R₂ est : 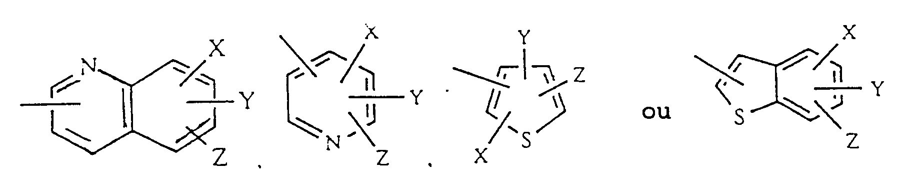 dans lesquels X, Y et Z ont, indépendamment, une valeur telle que définie pour X, Y et Z, ci-dessus, et sont identiques ou différents, ou
un sel pharmaceutiquement acceptable de celui-ci.

2. Composé suivant la revendication 1, de formule : 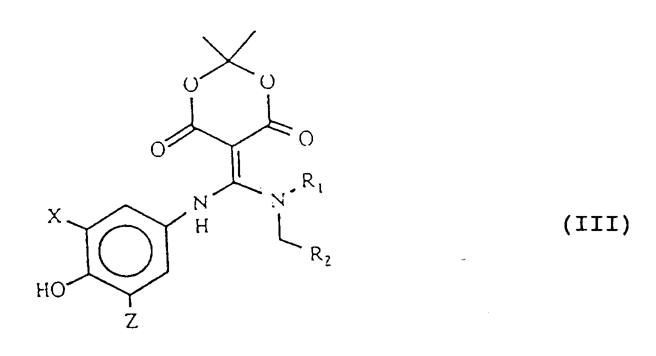 dans lequel :
X et Z sont, indépendamment, alcoyle en 1 à 6 atomes de carbone, branché en alpha;
R₁ est alcoyle en 1 à 18 atomes de carbone, et
R₂ est 2-, 3- ou 4-quinoléinyle, ou
un sel pharmaceutiquement acceptable de celui-ci.

3. Composé suivant la revendication 1, de formule : 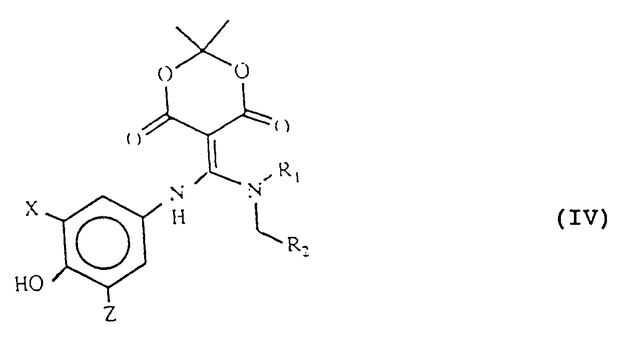 dans lequel :
X et Z sont, indépendamment, alcoyle en 1 à 6 atomes de carbone, branché en alpha;
R₁ est alcoyle en 1 à 18 atomes de carbone, et
R₂ est 2-, 3- ou 4-pyridinyle, ou un sel pharmaceutiquement acceptable de celui-ci.

4. Composé suivant la revendication 1, de formule : 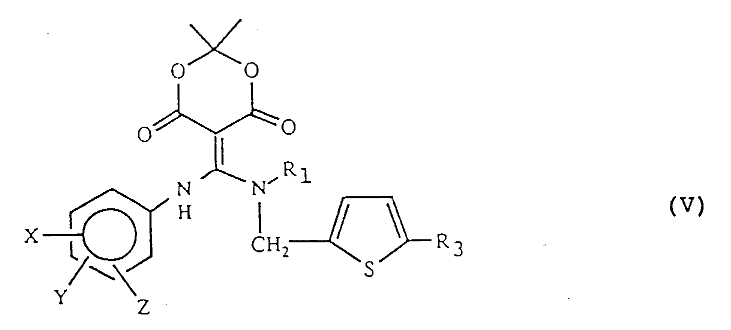 dans lequel :
X, Y et Z sont, indépendamment, hydrogène, alcoyle en 1 à 6 atomes de carbone, alcoxy en 1 à 6 atomes de carbone ou hydroxyle;
R₁ est alcoyle en 6 à 10 atomes de carbone ou cycloalcoyle en 5 à 7 atomes de carbone, et
R₃ est alcoyle en 1 à 6 atomes de carbone.

5. Composé suivant la revendication 1, de formule : 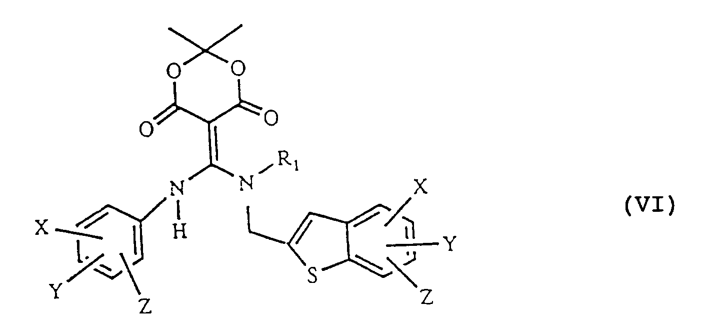 dans lequel :
X, Y et Z sont, indépendamment, alcoyle en 1 à 4 atomes de carbone, alcoxy en 1 à 4 atomes de carbone ou hydroxyle, et
R₁ est alcoyle en 1 à 18 atomes de carbone, ou
un sel pharmaceutiquement acceptable de celui-ci.

6. Composé suivant la revendication 1, qui est la 5-[[[3,5-bis(1,1-diméthyléthyl)-4-hydroxyphényl]amino]-[hexyl-(4-quinoléinylméthyl)amino]méthylène]-2,2-diméthyl-1,3-dioxanne-4,6-dione, ou un sel pharmaceutiquement acceptable de celle-ci.

7. Composé suivant la revendication 1, qui est la 5-[[[3,5-bis(1,1-diméthyléthyl)-4-hydroxyphényl]amino]-[hexyl-(3-quinoléinylméthyl)amino]méthylène]-2,2-diméthyl-1,3-dioxanne-4,6-dione, ou un sel pharmaceutiquement acceptable de celle-ci.

8. Composé suivant la revendication 1, qui est la 5-[(3,5-di-t-butyl-4-hydroxyphénylamino)[hexyl(pyridin-4-ylméthyl)amino]méthylène]-2,2-diméthyl-1,3-dioxanne-4,6-dione, ou un sel pharmaceutiquement acceptable de celle-ci.

9. Composé suivant la revendication 1, qui est la 5-[[[3,5-bis(1,1-diméthyléthyl)-4-hydroxyphényl]amino]-[[[5-(2,2-diméthylpropyl)-2-thiényl]méthyl]heptylamino]méthylène]-2,2-diméthyl-1,3-dioxanne-4,6-dione.

10. Composé suivant la revendication 1, qui est la 5-[[[3,5-bis(1,1-diméthyléthyl)-4-hydroxyphényl]amino]-[[[5-(2,2-diméthylpropyl)-2-thiényl]méthyl]-(1-méthylheptyl)amino]méthylène]-2,2-diméthyl-1,3-dioxanne-4,6-dione.

11. Composé suivant la revendication 1, qui est la 5-[[benzo[b]thiophén-2-ylméthyl-(1-méthylhexyl)amino]-(3,5-di-t-butyl-4-hydroxyphénylamino)méthylène]-2,2-diméthyl-1,3-dioxanne-4,6-dione, ou un sel pharmaceutiquement acceptable de celle-ci.

12. Procédé de préparation d'un composé de formule (I) tel que défini à la revendication 1, ou un sel pharmaceutiquement acceptable de celui-ci, qui comprend :
a) la réaction d'un composé de formule : 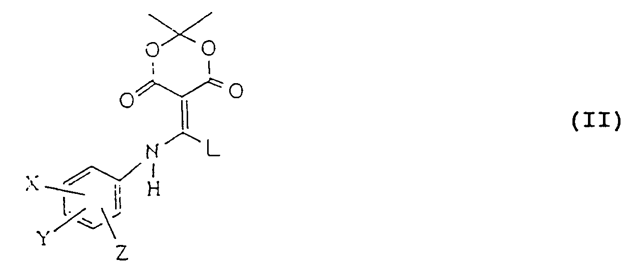 dans lequel :
X, Y et Z sont tels que définis ci-dessus, et
L est un groupe partant, avec une amine de formule :
HN(R₁)CH₂R₂
dans laquelle :
R₁ et R₂ sont tels que définis à la revendication 1, ou
b) la réaction de la 2,2-diméthyl-1,3-dioxanne-4,6-dione (acide de Meldrum) avec un carbodiimide de formule : 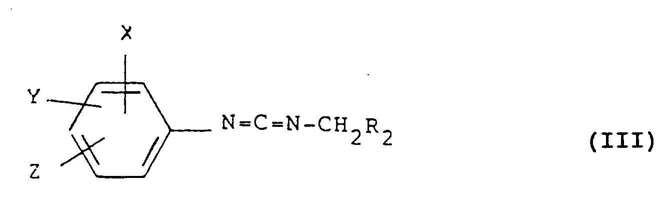 dans lequel :
R₂ est tel que défini à la revendication 1, pour donner un composé de formule (I) tel que défini ci-dessus, dans lequel R₁ est hydrogène;
et, si désiré, après le procédé a) ou b), la conversion de toute valeur X, Y ou Z en l'une des autres valeurs pour X, Y et Z, par des procédés connus et ensuite, si désiré, isolement du produit sous forme d'un sel pharmaceutiquement acceptable.

13. Composition pharmaceutique comprenant un composé de formule (I) ou un sel pharmaceutiquement acceptable de celui-ci, tel que défini dans l'une quelconque des revendications 1 à 11, et un excipient pharmaceutiquement acceptable.

## Revendications (Revendications pour l'(les) Etat(s) contractant(s) suivant(s): ES, GR)

1. Procédé de préparation d'un composé de formule : 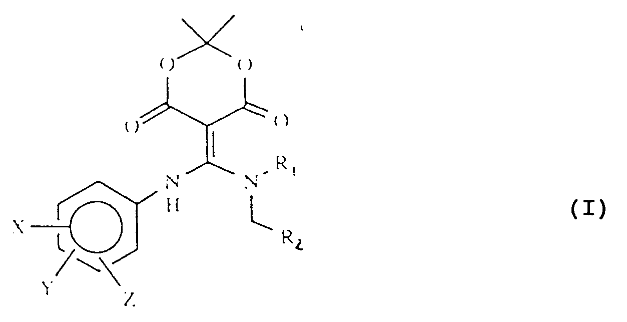 dans lequel :
X, Y et Z sont, indépendamment, hydrogène, halogène, hydroxyle, nitro, cyano, carboxyle, trifluorométhyle, phényle, amino, alcoylamino en 1 à 12 atomes de carbone, dialcoylamino dans lequel chaque radical alcoyle comprend de 1 à 12 atomes de carbone, alcoyle en 1 à 12 atomes de carbone ou alcoxy en 1 à 12 atomes de carbone;
R₁ est hydrogène, alcoyle en 1 à 18 atomes de carbone, alcényle en 2 à 18 atomes de carbone, cycloalcoyle en 5 à 8 atomes de carbone, phényle, benzyle ou phényle ou benzyle substitué, où les substituants sont alcoyle en 1 à 12 atomes de carbone ou alcoxy en 1 à 12 atomes de carbone;
R₂ est : 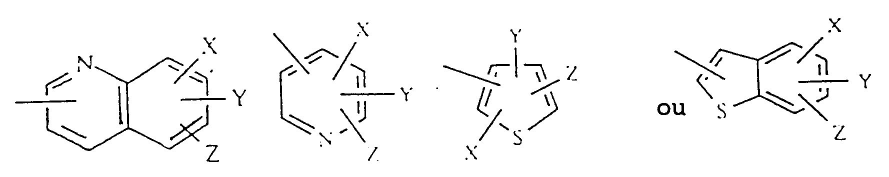 dans lesquels X, Y et Z ont, indépendamment, une valeur telle que définie pour X, Y et Z, ci-dessus, et sont identiques ou différents, ou
un sel pharmaceutiquement acceptable de celui-ci, qui comprend :
a) la réaction d'un composé de formule : 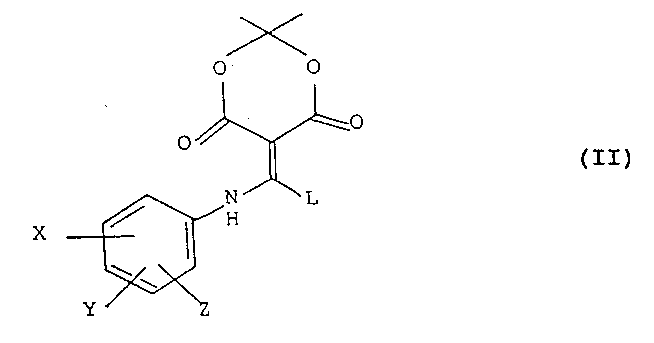 dans lequel :
X, Y et Z sont tels que définis ci-dessus, et
L est un groupe partant, avec une amine de formule :
HN(R₁)CH₂R₂
dans laquelle :
R₁ et R₂ sont tels que définis ci-dessus, ou
b) la réaction de la 2,2-diméthyl-1,3-dioxanne-4,6-dione (acide de Meldrum) avec un carbodiimide de formule : 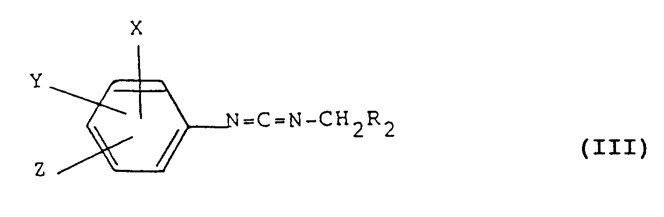 dans lequel :
R₂ est tel que défini ci-dessus, pour donner un composé de formule (I) tel que défini ci-dessus, dans lequel R₁ est hydrogène;
et, si désiré, après le procédé a) ou b), la conversion de toute valeur X, Y ou Z en l'une des autres valeurs pour X, Y et Z, par des procédés connus et ensuite, si désiré, isolement du produit sous forme d'un sel pharmaceutiquement acceptable.

2. Procédé suivant la revendication 1, dans lequel le composé préparé a la formule : 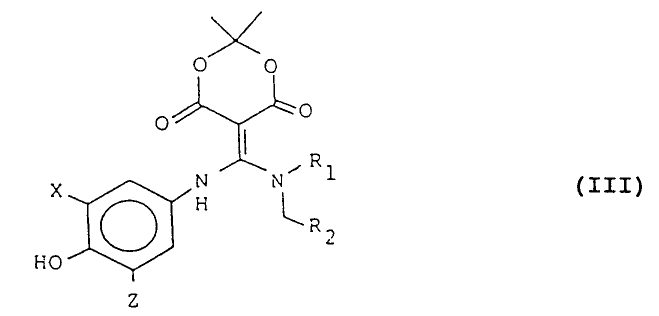 dans lequel :
X et Z sont, indépendamment, alcoyle en 1 à 6 atomes de carbone, branché en alpha;
R₁ est alcoyle en 1 à 18 atomes de carbone, et
R₂ est 2-, 3- ou 4-quinoléinyle, ou
un sel pharmaceutiquement acceptable de celui-ci.

3. Procédé suivant la revendication 1, dans lequel le composé préparé a la formule : 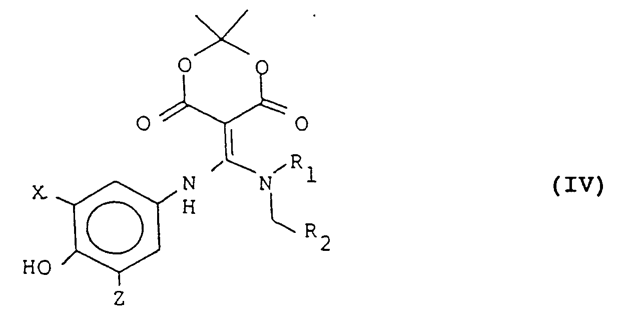 dans lequel :
X et Z sont, indépendamment, alcoyle en 1 à 6 atomes de carbone, branché en alpha;
R₁ est alcoyle en 1 à 18 atomes de carbone, et
R₂ est 2-, 3- ou 4-pyridinyle, ou
un sel pharmaceutiquement acceptable de celui-ci.

4. Procédé suivant la revendication 1, dans lequel le composé préparé a la formule : 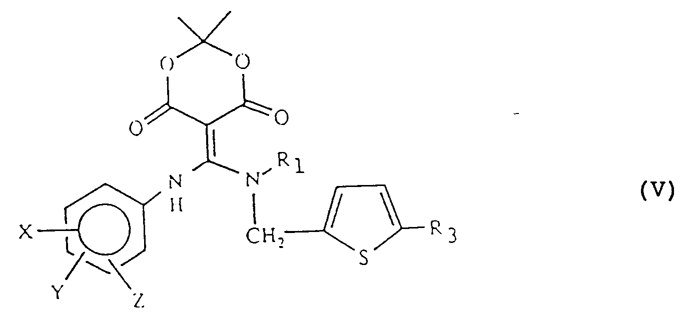 dans lequel :
X, Y et Z sont, indépendamment, hydrogène, alcoyle en 1 à 6 atomes de carbone, alcoxy en 1 à 6 atomes de carbone ou hydroxyle;
R₁ est alcoyle en 6 à 10 atomes de carbone ou cycloalcoyle en 5 à 7 atomes de carbone, et
R₃ est alcoyle en 1 à 6 atomes de carbone.

5. Procédé suivant la revendication 1, dans lequel le composé préparé a la formule : 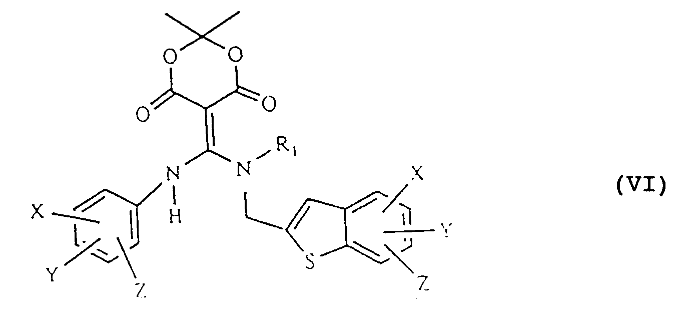 dans lequel :
X, Y et Z sont, indépendamment, alcoyle en 1 à 4 atomes de carbone, alcoxy en 1 à 4 atomes de carbone ou hydroxyle, et
R₁ est alcoyle en 1 à 18 atomes de carbone, ou
un sel pharmaceutiquement acceptable de celui-ci.

6. Procédé suivant la revendication 1, dans lequel le composé préparé est la 5-[[[3,5-bis(1,1-diméthyléthyl)-4-hydroxyphényl]amino][hexyl-(4-quinoléinylméthyl)amino]méthylène]-2,2-diméthyl-1,3-dioxanne-4,6-dione, ou un sel pharmaceutiquement acceptable de celle-ci.

7. Procédé suivant la revendication 1, dans lequel le composé préparé est la 5-[[[3,5-bis(1,1-diméthyléthyl)-4-hydroxyphényl]amino][hexyl-(3-quinoléinylméthyl)-amino]méthylène]-2,2-diméthyl-1,3-dioxanne-4,6-dione, ou un sel pharmaceutiquement acceptable de celle-ci.

8. Procédé suivant la revendication 1, dans lequel le composé préparé est la 5-[(3,5-di-t-butyl-4-hydroxyphénylamino)[hexyl(pyridin-4-ylméthyl)amino]méthylène]-2,2-diméthyl-1,3-dioxanne-4,6-dione, ou un sel pharmaceutiquement acceptable de celle-ci.

9. Procédé suivant la revendication 1, dans lequel le composé préparé est la 5-[[[3,5-bis(1,1-diméthyléthyl)-4-hydroxyphényl]amino]-[[[5-(2,2-diméthylpropyl)-2-thiényl]méthyl]heptylamino]méthylène]-2,2-diméthyl-1,3-dioxanne-4,6-dione.

10. Procédé suivant la revendication 1, dans lequel le composé préparé est la 5-[[[3,5-bis(1,1-diméthyléthyl)-4-hydroxyphényl]amino]-[[[5-(2,2-diméthylpropyl)-2-thiényl]méthyl]-(1-méthylheptyl)amino]méthylène]-2,2-diméthyl-1,3-dioxanne-4,6-dione.

11. Procédé suivant la revendication 1, dans lequel le composé préparé est la 5-[[benzo[b]thiophén-2-ylméthyl-(1-méthylhexyl)amino]-(3,5-di-t-butyl-4-hydroxyphénylamino)méthylène]-2,2-diméthyl-1,3-dioxanne-4,6-dione, ou un sel pharmaceutiquement acceptable de celle-ci.

12. Procédé de préparation d'une composition pharmaceutique qui comprend la mise en association d'un composé de formule (I) ou d'un sel pharmaceutiquement acceptable de celui-ci, tel que défini dans l'une quelconque des revendications 1 à 11, avec un excipient pharmaceutiquement acceptable.
